(19) Europäisches Patentamt
European Patent Office
Office européen des brevets

(11) EP 2 136 790 B1

(12) EUROPÄISCHE PATENTSCHRIFT

(45) Veröffentlichungstag und Bekanntmachung des Hinweises auf die Patenterteilung:
**16.05.2012 Patentblatt 2012/20**

(21) Anmeldenummer: **08736228.1**

(22) Anmeldetag: **15.04.2008**

(51) Int Cl.:
***A61K 9/16*** *(2006.01)* ***A61K 9/28*** *(2006.01)*
***A61K 36/00*** *(2006.01)*

(86) Internationale Anmeldenummer:
**PCT/EP2008/054536**

(87) Internationale Veröffentlichungsnummer:
**WO 2008/128924 (30.10.2008 Gazette 2008/44)**

# (54) PRÄPARATE ZUR GESTEUERTEN FREISETZUNG VON BIOAKTIVEN NATURSTOFFEN

PREPARATION FOR THE CONTROLLED RELEASE OF BIOACTIVE NATURAL SUBSTANCES

PRÉPARATIONS POUR LA LIBÉRATION CONTRÔLÉE DE SUBSTANCES NATURELLES BIOACTIVES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **18.04.2007 EP 07106432**

(43) Veröffentlichungstag der Anmeldung:
**30.12.2009 Patentblatt 2009/53**

(73) Patentinhaber: **Evonik Degussa GmbH**
**45128 Essen (DE)**

(72) Erfinder:
- **SEILER, Matthias**
  **64347 Griesheim (DE)**
- **HANEKE, Martin**
  **37696 Marienmünster (DE)**
- **MARCKMANN, Henning**
  **86807 Buchloe (DE)**
- **PILZ, Stephan**
  **70736 Fellbach (DE)**
- **IRFAN, Muhammad**
  **63457 Hanau (DE)**
- **KLEE-LAQUAI, Saskia**
  **40721 Hilden (DE)**
- **HILLS, Geoffrey**
  **45355 Essen (DE)**
- **FARWICK, Mike**
  **45138 Essen (DE)**
- **LERSCH, Peter**
  **46537 Dinslaken (DE)**
- **KOBUS, Axel**
  **63225 Langen (DE)**

(56) Entgegenhaltungen:
**WO-A-93/17060 WO-A-96/12754**
**FR-A- 2 787 729 US-A1- 2004 197 416**
**US-B1- 6 379 683**

- **JIANHUA ZOU ET AL.: "Encapsulation and Controlled Release of a Hydrophobic Drug Using a Novel Nanoparticle-Forming Hyperbranched Polyester" MACROMOL. BIOSCI., Bd. 5, 2005, Seiten 662-668, XP002461317**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Präparate zur gesteuerten Freisetzung von bioaktiven Naturstoffen sowie Verfahren zur Herstellung und Verwendung dieser Präparate.

Viele bioaktive Naturstoffe, wie beispielsweise Pflanzenextrakte, Vitamine oder Öle, die einen hohen Anteil an ungesättigten Fettsäuren enthalten, sind sehr oxidationsempfindlich und müssen daher bei tiefen Temperaturen oder unter Inertgasatmosphäre gelagert werden. Diese Lagerungsbedingungen können jedoch vielfach nur sehr bedingt eingehalten werden. Aus diesem Grund werden viele dieser Substanzen in Präparate überführt, die eine einfache Lagerung ermöglichen. Des Weiteren erlauben diese Präparate vielfach eine gezielte Freisetzung dieser Wirkstoffe an einem vorgesehenen Ort oder über eine bestimmte Zeit.

Im Allgemeinen werden diese Präparate vielfach als verkapselte Systeme beschrieben, die einen Wirkstoff und ein Verkapselungsmaterial umfassen, das eine Schutzfunktion ausübt oder über dessen Eigenschaften das Freisetzungsprofil gezielt gesteuert werden kann.

Als Verkapselungsmaterial werden vielfach Polymere, beispielsweise hyperverzweigte Polymere eingesetzt. Die Verwendung von hyperverzweigten Polymeren als Trägerstoff für Arzneimittel wird beispielsweise in WO 2004/072153 dargelegt. Gemäß dieser Druckschrift ermöglicht das Trägermolekül eine retardierte Freisetzung sowie einen erleichterten Transport der Arzneistoffe in die Zellen. In diesem Zusammenhang werden insbesondere modifizierte Dendrimere dargelegt, die Stickstoff umfassende Gruppen aufweisen.

Des Weiteren beschreibt die Druckschrift WO 00/065024 Polymere zur Verkapselung von hydrophoben Molekülen. Hierbei wird eine Vielzahl von hydrophoben Resten an ein Polyolkern gebunden, wobei das erhaltene Polymer anschließend durch Polyalkylenoxide umgesetzt wird, um ein wasserlösliches Polymer zu erhalten.

Des Weiteren beschreibt die Druckschrift WO 2005/034909 Zusammensetzungen umfassend ein hyperverzweigtes Polymer, welches an einen biologisch aktiven Rest gekoppelt ist.

Darüber hinaus beschreibt die Druckschrift WO 03/037383 Präparate, die hyperverzweigte Polymere umfassen. Als hyperverzweigte Polymere werden insbesondere Polyamidoamine oder Polypropylenamine dargelegt.

Des Weiteren werden hyperverzweigte Polymere in der Druckschrift WO 00/06267 beschrieben, wobei als hyperverzweigte Polymere insbesondere Polyetherimide dargelegt werden.

Weiterhin werden Präparate, die dendritische Polymere und pharmazeutische Wirkstoffe umfassen, in WO 03/033027 dargelegt, wobei das Dendrimer katonische Gruppen umfasst.

Des Weiteren wird die Verwendung von hyperverzweigten Polymeren zur gezielten Freisetzung von Wirkstoffen von Zou et al. Macromol. Biosci. 5 (2005) 662-668 beschrieben. Hierbei werden hyperverzweigte Polymere mit ionischen Gruppen versehen.

Weiterhin werden in den Druckschriften US 6,379,683 bzw. EP 1 034 839 B1 Nanokapseln beschrieben, die hyperverzweigte Polymere aufweisen. Nachteilig ist hierbei insbesondere, dass die Nanokapseln nicht aus der Dispersion isoliert und weiterverarbeitet werden können. Darüber hinaus werden zur Herstellung der Nanokapseln organische Lösungsmittel eingesetzt, die vielfach nicht vollständig aus der Dispersion entfernt werden können.

Das Dokument US 6,432,423 beschreibt hyperverzweigte Polymere, die Polyestereinheiten umfassen können. Diese Polymere dienen insbesondere als Filmbildner in kosmetischen Zusammensetzungen, die Fette enthalten können. Allerdings wird der Schmelzpunkt der Fette nicht beschrieben. Des Weiteren wird dieses Polymer lediglich als Bestandteil einer herkömmlichen Mischung dargelegt, ohne dass hierdurch eine gesteuerte Freisetzung der Wirkstoffe erzielt wird.

Polymere, die den in US 6,432,423 beschriebenen Polymeren ähnlich sind, werden des Weiteren in US 6,475,495 dargelegt. Gemäß dieser Druckschrift können diese Polymere insbesondere als Geliermittel in kosmetischen Zusammensetzungen eingesetzt werden. Allerdings werden keine Präparate beschrieben, die eine gesteuerte Freisetzung von Wirkstoffen ermöglichen.

Kosmetische Zusammensetzungen, die hyperverzweigte Polymere umfassen, werden weiterhin in US 2006/0030686 dargelegt. Hierbei dienen diese Polymere insbesondere als Formulierungshilfsmittel. Präparate, die eine gesteuerte Wirkstofffreisetzung erlauben, werden jedoch nicht beschrieben.

Präparate, durch die eine gesteuerte Freisetzung von Wirkstoffen erzielt wird, sind beispielsweise Gegenstand der Druckschrift WO 2006/047714. Diese Präparate umfassen insbesondere einen an ein Polymer oder Oligomer kovalent gebundenen Wirkstoff. Die Freisetzung des Wirkstoffes erfolgt insbesondere durch eine enzymatische Spaltung dieser kovalenten Bindung. Hierdurch kann vorteilhaft der Wirkstoff gezielt an einer vorgegebenen Wirkungsstelle freigesetzt werden. Nachteilig an diesen Systemen ist jedoch, dass der Wirkstoff zunächst an ein Polymer gebunden werden muss. Dies schränkt die Anwendung dieses Verfahrens ein. Weiterhin wird hierdurch die Lagerfähigkeit der Wirkstoffe nur unwesentlich verbessert. Darüber hinaus können naturnahe Wirkstoffkombinationen, beispielsweise Pflanzenextrakte nur sehr schwer in natürlicher Zusammensetzung in ein entsprechendes Präparat überführt werden.

Die Druckschrift WO 2004/028269 beschreibt Polymere, die in biologisch abbaubaren Kaugummis eingesetzt werden können. Diese Polymere, die insbesondere Polyestereinheiten umfassen können, weisen einen bestimmten Verzweigungsgrad auf. Die gesteuerte Freisetzung von Wirkstoffen ist jedoch nicht Gegenstand dieser Druckschrift.

Die Dissertation von S. Suttiruengwong "Silica Aerogels and Hyperbranched Polymers as Drug Delivery Systems", Erlangen 2005, beschreibt verkapselte Systeme, die hyperverzweigte Polymere aufweisen können.

Darüber hinaus beschreibt das Dokument US 2004/016394 kosmetische Zusammensetzungen, die hyperverzweigte Polymere umfassen können. Allerdings werden die Eigenschaften dieser hyperverzweigten Polymere, insbesondere das Molekulargewicht, der Verzweigungsgrad, die Hydroxyzahl oder der Schmelzpunkt nicht explizit aufgeführt.

Dementsprechend ist festzuhalten, dass viele Präparate bekannt sind, die ein Verkapselungsmaterial, beispielsweise ein hyperverzweigtes Polymer, und einen Wirkstoff umfassen. Jedoch besteht das dauerhafte Bestreben möglichst vorteilhafte Präparate zur Verfügung zu stellen. Des Weiteren ist der Aufwand zur Herstellung der zuvor beschriebenen hyperverzweigten Polymere relativ hoch. Dementsprechend sind diese Polymere vergleichsweise teuer.

Darüber hinaus ist die Handhabung der Präparate des Standes der Technik schwierig, da diese vielfach als Dispersionen vorliegen und hieraus nicht isoliert werden können. Dementsprechend können diese Präparate nur in einer sehr begrenzten Weise und technisch anspruchsvoll in Endprodukte eingearbeitet werden.

Weiterhin werden zur Herstellung der zuvor dargelegten Präparate des Standes der Technik vielfach organische Lösungsmittel eingesetzt. Der Endverbraucher wünscht jedoch naturnahe Produkte, die keinen Restgehalt an diesen Verbindungen aufweisen.

In Anbetracht des hierin angegebenen und diskutierten Standes der Technik war es eine Aufgabe der vorliegenden Erfindung, Präparate zur Verfügung zu stellen, die ein hervorragendes Eigenschaftsprofil aufweisen.

Das Eigenschaftsprofil umfasst insbesondere die Steuerungsmöglichkeiten der Freisetzung des bioaktiven Naturstoffs. Gemäß einem Aspekt der vorliegenden Erfindung sollten die erfindungsgemäßen Präparate den bioaktiven Naturstoff in einem gewählten Medium über einen möglichst langen Zeitraum freisetzen können, wobei die Freisetzungsrate vorzugsweise konstant bleiben sollte. Gemäß einer weiteren Ausführungsform der vorliegenden Erfindung sollte die Freisetzung gezielt innerhalb einer kurzen Zeitspanne erfolgen. Hierbei kann eine Aufgabe darin gesehen werden, ein Präparat zur Verfügung zu stellen, bei dem die Freisetzung des Wirkstoffes möglichst einfach und zuverlässig gesteuert werden kann.

Weiterhin war es Aufgabe der vorliegenden Erfindung Präparate zur Verfügung zu stellen, die einen besonders hohen Gehalt an bioaktiven Naturstoff umfassen.

Des Weiteren sollte das Präparat eine besonders hohe Stabilität zeigen, wodurch insbesondere empfindliche Naturstoffe über einen besonders langen Zeitraum gelagert werden können, ohne dass die Eigenschaften des Naturstoffs wesentlich geändert werden. Hierbei sollten die Präparate ebenfalls eine hohe Scherstabilität aufweisen, so dass eine einfache und problemlose Verarbeitung der Präparate möglich ist.

Weiterhin war es eine Aufgabe Präparate zur Verfügung zu stellen, die einfach und kostengünstig hergestellt werden können.

Gelöst werden diese Aufgaben sowie weitere, die zwar nicht explizit genannt werden, sich aber aus den hierin diskutierten Zusammenhängen wie selbstverständlich ableiten lassen oder sich aus diesen zwangsläufig ergeben, durch die in Anspruch 1 beschriebenen Präparate. Zweckmäßige Abwandlungen dieser Präparate werden in den auf Anspruch 1 rückbezogenen Unteransprüchen unter Schutz gestellt.

Gegenstand der vorliegenden Erfindung ist dementsprechend ein Präparat umfassend ein Verkapselungsmaterial und mindestens einen bioaktiven Naturstoff, wobei der bioaktive Naturstoff gesteuert aus dem Präparat freigesetzt werden kann, welches dadurch gekennzeichnet ist, dass das Verkapselungsmaterial mindestens ein Glycerid mit einem Schmelzpunkt von mindestens 35°C, das von Carbonsäuren mit 12 bis 24 Kohlnstattatomen abgeleitet ist und zusätzlich mindestens ein Polymer mit Polyestereinheiten umfasst, wobei das Polymer mit Polyestereinheiten eine Schmelztemperatur von mindestens 35°C und eine Viskosität im Bereich von 50 mPa*s bis 250 Pa*s, gemessen mittels Rotationsviskosimetrie bei 110°C, aufweist.

Durch die erfindungsgemäßen Maßnahmen gelingt es überraschend, Präparate mit einem hervorragenden Eigenschaftsprofil zur Verfügung zu stellen, die besonders einfach und kostengünstig, insbesondere ohne Verwendung von organischen Lösungsmitteln erhalten werden können.

Die erfindungsgemäßen Präparate können insbesondere ein hervorragendes Freisetzungsprofil des bioaktiven Naturstoffs aufweisen, wobei sowohl eine Freisetzung über einen besonders langen Zeitraum als auch eine kurzzeitige Freisetzung nach Betätigung eines Auslösemechanismus realisiert werden können.

Darüber hinaus können die Präparate eine hohe Scherstabilität aufweisen. Hierdurch können die erfindungsgemäß erhältlichen Präparate besonders einfach und problemlos verarbeitet werden. Ferner können die Präparate besonders einfach auf bestimmte Bedürfnisse abgestimmt werden. So können Präparate die unterschiedlichsten Freisetzungsmechanismen aufweisen. Hierzu gehören unter anderem Mechanismen, die auf einem enzymatischen Abbau des Verkapselungsmaterials oder einer pH-selektiven Öffnung des Verkapselungsmaterials basieren; temperatur- bzw. lösungsmittelgesteuerte Vorgänge, Einwirkung von Energie auf die Präparate, zum Beispiel Bestrahlung von Partikeln mit elektromagnetischer Strahlung, Bestrahlung mit Ultraschall und/oder Einwirkung von Scherkräften. Darüber hinaus können die Präparate einen hohen Anteil an bioaktiven Naturstoff aufweisen.

Weiterhin ermöglicht das vorliegend dargelegte Präparat eine besonders stabile Aufbewahrung von empfindlichen Naturstoffen. Dementsprechend können empfindliche Substanzen sicher und einfach gelagert werden.

Darüber hinaus sind die erfindungsgemäßen Präparate überraschend stabil, so dass diese über einen langen Zeitraum aufbewahrt werden können, ohne dass ein Abbau erfolgt. Des Weiteren können die Präparate aufgrund der hohen Scherstabilität problemlos verarbeitet werden. Ein besonderer Vorteil ergibt sich insbesondere daraus, dass die Präparate bei Raumtemperatur, insbesondere bei 25°C, fest sind, so dass dieser Feststoff ohne aufwendige und schwierig zu beherrschende Verfahrensschritte in viele Endprodukte eingearbeitet werden kann.

Die Präparate der vorliegenden Erfindung können vielfach besonders einfach und kostengünstig erhalten werden. Des Weiteren sind die erfindungsgemäßen Präparate gesundheitlich unbedenklich. Hierbei ist insbesondere von Vorteil, dass die erfindungsgemäßen Präparate ohne die Verwendung von organischen Lösungsmitteln erhältlich sind. Daher können sehr naturnahe Präparate erhalten werden, die keinen Restgehalt an organischen Lösungsmitteln enthalten.

Der Begriff "Verkapselungsmaterial" bezeichnet insbesondere eine Mischung, die mindestens ein Glycerid mit einem Schmelzpunkt von mindestens 35°C und zusätzlich mindestens ein Polymer mit Polyestereinheiten umfasst, wobei das Polymer mit Polyestereinheiten eine Schmelztemperatur von mindestens 35°C und eine Viskosität im Bereich von 50 mPa*s bis 100 Pa*s aufweist. Das Verkapselungsmaterial dient insbesondere zum Schutz des bioaktiven Naturstoffs. Dementsprechend zeigt das Verkapselungsmaterial vorzugsweise eine hohe Stabilität gegen Oxidation. Von besonderem Interesse sind hierbei insbesondere Materialien, die enzymatisch abbaubar sind. Hierdurch gelingt es insbesondere einen Mechanismus bereitzustellen, der eine gezielte Freisetzung der Wirkstoffe auf menschlicher Haut ermöglicht.

Das erfindungsgemäß einzusetzende Verkapselungsmaterial umfasst mindestens ein Glycerid, das einen Schmelzpunkt von mindestens 35°C, vorzugsweise mindestens 40°C aufweist. Von besonderem Interesse sind insbesondere Glyceride, die einen Schmelzpunkt im Bereich von 45 bis 80°C, besonders bevorzugt 50 bis 65 °C aufweisen.

Zweckmäßige Ausführungsformen des erfindungsgemäß einzusetzenden Verkapselungsmaterials umfassen insbesondere ein Glycerid, das eine dynamische Viskosität im Bereich von 5 bis 200 mPa*s, vorzugsweise 10 bis 50 mPa*s und besonders bevorzugt 15 bis 22 mPa*s bei 70°C aufweist, gemessen gemäß DIN EN ISO 3219.

Der Begriff "Glycerid" bezeichnet vorliegend einen Ester einer Carbonsäure mit Glycerin (Propan-1,2,3-triol). Bevorzugt können zwei oder drei der Hydroxygruppen des Glycerins mit zwei oder drei Carbonsäuren verestert sein, die 12 bis 24 und besonders bevorzugt 14 bis 20 Kohlenstoffatome aufweisen. Von besonderem Interesse sind insbesondere Triglycerinester, die drei Gruppen aufweisen, die von Carbonsäuren mit 12 bis 24, besonders bevorzugt 14 bis 20 Kohlenstoffatomen abgeleitet sind.

Bevorzugte Triglycerinester weisen insbesondere die Formel (I) auf, worin die Reste R1, R2 und R3 unabhängig einen Kohlenwasserstoffrest mit 11 bis 23 und besonders bevorzugt 13 bis 19 Kohlenstoffatomen bedeuten.

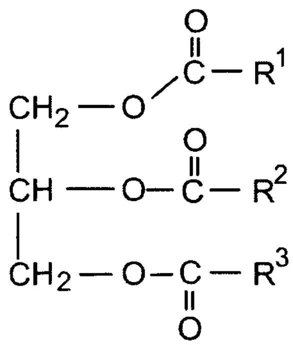

(I)

Kohlenwasserstoffreste bezeichnen vorliegend insbesondere gesättigte und/oder ungesättigte Reste, die vorzugsweise aus Kohlenstoff und Wasserstoff bestehen. Diese Reste können cyclisch, linear oder verzweigt sein. Hierzu gehören insbesondere Alkylreste und Alkenylreste, wobei die Alkenylreste eine, zwei, drei oder mehr Kohlenstoff-Kohlenstoff-Doppelbindungen umfassen können.

Zu den bevorzugten Alkylresten gehören insbesondere die Undecyl-, Dodecyl-, Tridecyl-, Tetradecyl-, Pentadecyl-, Hexadecyl-, Heptadecyl-, Octadecyl-, Nonadecyl-, Eicosyl-Gruppe.

Beispiele für Alkenylreste mit einer Kohlenstoff-Kohlenstoff-Doppelbindung sind unter anderem die Undecenyl-, Dodecenyl-, Tridecenyl-, Tetradecenyl-, Pentadecenyl-, Hexadecenyl-, Heptadecenyl-, Octadecenyl-, Nonadecenyl-, Eicosenyl-Gruppe.

Die zuvor dargelegten Kohlenwasserstoffreste können Substituenten und/oder Heteroatome aufweisen. Hierzu gehören insbesondere Gruppen, die Sauerstoff, Stickstoff und/oder Schwefel umfassen, wie beispielsweise Hydroxygruppen, Thiolgruppen oder Aminogruppen. Der Anteil dieser Gruppen sollte jedoch so gering sein, dass die Eigenschaften der Glyceride nicht nachteilig beeinflusst werden.

Zu den bevorzugten gesättigten Carbonsäuren, von denen die Glyceride abgeleitet sind, gehören unter anderem , Dodecansäure, Tetradecansäure, Hexadecansäure, Heptadecansäure, Octadecansäure, Eicosansäure, Docosansäure, Tetracosansäure, besonders bevorzugt Eicosansäure und Docosansäure.

Die Eigenschaften der Glyceride sind insbesondere von der Art und der Menge der Fettsäuren abhängig, die in den Glyceriden enthalten sind. Dementsprechend ist der Anteil an den langkettigen, gesättigten Fettsäuren im Vergleich zu den Anteilen, die in vielen natürlich vorkommenden Ölen und Fetten vorliegen, sehr hoch. Erfindungsgemäß kann ein Monoglycerid eingesetzt werden, das durch Veresterung von Glycerin mit einer besonders langkettigen Fettsäure erhalten wurde. Des weiteren können Di- oder Triglyceride verwendet werden, die einen entsprechend hohen Anteil an Carbonsäuren, die 12 bis 24 Kohlenstoffatome aufweisen.

Von besonderem Interesse sind insbesondere Triglyceride, die einen hohen Anteil an Stearinsäure und/oder Palmitinsäure aufweisen. Vorzugsweise können insbesondere Triglyceride eingesetzt werden, die mindestens 10 Gew.-%, bevorzugt mindestens 20 Gew.-% und ganz besonders bevorzugt mindestens 30 Gew.-% an Stearinsäure- und/oder Palmitinsäurereste, bezogen auf den Gesamtgehalt an Fettsäuren, aufweisen. Gemäß einer bevorzugten Ausführungsform werden insbesondere Triglyceride eingesetzt, die Stearinsäure- und Palmitinsäuregruppen umfassen. Das Gewichtsverhältnis von Stearinsäure zu Palmitinsäure liegt vorzugsweise im Bereich von 10:1 bis 1:1, besonders bevorzugt 4:1 bis 1,5:1. Gemäß einer besonders zweckmäßigen Ausführungsform kann insbesondere ein Triglycerid eingesetzt werden, dessen Fettsäurespektrum vorzugsweise 50 bis 90 Gew.-%, besonders bevorzugt 60 bis 80 Gew.-% und ganz besonders bevorzugt etwa 70 Gew.-% Stearinsäure und vorzugsweise 10 bis 50 Gew.-%, besonders bevorzugt 20 bis 40 Gew.-% und ganz besonders bevorzugt etwa 30 Gew.-% Palmitinsäure umfasst.

Die Oxidationsstabilität der als Verkapselungsmaterial eingesetzten Glyceride ist unter anderem von dem Anteil an ungesättigten Carbonsäuren abhängig. Je geringer dieser Anteil, desto höher die Oxidationsstabilität. Ein bevorzugt einzusetzendes Glycerid weist vorzugsweise eine Iodzahl kleiner oder gleich 50, besonders kleiner oder gleich 20 und besonders bevorzugt kleiner oder gleich 10 auf. Die Iodzahl kann insbesondere gemäß DIN EN 14111 bestimmt werden.

Neben einer Synthese der zuvor dargelegten Glyceride durch Veresterung von Glycerin mit entsprechenden Carbonsäuren können diese Glyceride auch aus natürlichen Quellen, insbesondere aus Pflanzen gewonnen werden. So können insbesondere Triglyceride eingesetzt werden, die kommerziell von Goldschmidt GmbH, Essen unter der Handelsbezeichnung Tegin® erhältlich sind. Darüber hinaus können geeignete Triglyceride eingesetzt werden, die von der Fa. Cognis GmbH & Co. KG unter der Bezeichnung Edenor® erhältlich sind, wobei insbesondere Edenor® NHTi V verwendet werden kann. Das erfindungsgemäß als Verkapselungsmaterial einzusetzende Glycerid unterscheidet sich von den freizusetzenden bioaktiven Naturstoffen. Im Allgemeinen sind die freizusetzenden Naturstoffe oxidationsempfindlich oder auf sonstige Weise instabil. Dementsprechend kann das als Verkapselungsmaterial einzusetzende Glycerid von dem bioaktiven Naturstoff durch die Oxidationsempfindlichkeit, die beispielsweise durch die Iodzahl (Gramm Iod, das an die Doppelbindungen von 100g Naturstoff angelagert werden kann) bestimmt werden kann, unterschieden werden. Von besonderem Interesse sind insbesondere Präparate, deren bioaktiver Naturstoff eine Iodzahl aufweist, die um mindestens 5 größer ist, als die Iodzahl des als Verkapselungsmaterial einzusetzenden Glycerids. Bevorzugt beträgt diese Iodzahldifferenz mindestens 10, besonders bevorzugt mindestens 20.

Das Molekulargewicht der bevorzugt als Verkapselungsmaterial einzusetzenden Glyceride ist an sich nicht kritisch. Im Allgemeinen werden vielfach Glyceride mit einem Molekulargewicht im Bereich von 200 bis 1600 g/mol, vorzugsweise 400 bis 1500 g/mol und besonders bevorzugt 500 bis 1200 g/mol eingesetzt.

Der Anteil an Glycerid, bezogen auf das Gewicht des Präparats, liegt vorzugsweise im Bereich von 1 bis 99,5 Gew.-%, besonders bevorzugt 10 bis 80 Gew.-% und ganz besonders bevorzugt 20 bis 70 Gew.-%.

Das Verkapselungsmaterial umfasst neben mindestens einem Glycerid zusätzlich ein Polymer, das Polyestereinheiten umfasst. Der Begriff "Polymer mit Polyestereinheiten" bezeichnet im Rahmen der vorliegenden Erfindung eine makromolekulare Verbindung, die Einheiten umfasst, die Polyester bilden können. Hierzu gehören insbesondere Einheiten, die von Diolen und Dicarbonsäuren abgeleitet sind, und Einheiten, die von Verbindungen mit mindestens einer Carbonsäuregruppe und mindestens einer Hydroxygruppe abgeleitet sind. Der Anteil an Einheiten, die Polyester bilden können, bezogen auf das Gewicht des Polymeren, beträgt vorzugsweise mindestens 10 Gew.-%, besonders bevorzugt mindestens 30 Gew.-%.

Das Polymer mit Polyestereinheiten weist eine Schmelztemperatur von mindestens 30°C, vorzugsweise mindestens 35°C auf. Zweckmäßig sind insbesondere Polymere mit Polyestereinheiten, die eine Schmelztemperatur Bereich von 30°C bis 90°C, besonders bevorzugt 35°C bis 70°C und ganz besonders bevorzugt 40°C bis 65°C aufweisen. Die Schmelztemperatur kann mittels Differential Scanning Calometry (DSC) bestimmt werden, z.B. mit dem Apparat Mettler DSC 27 HP und einer Heizrate von 10°C/min.

Das im Verkapselungsmaterial enthaltene Polymer mit Polyestereinheiten weist eine Viskosität im Bereich von 50 mPa*s bis 250 Pa*s, bevorzugt im Bereich von 100 mPa*s bis 100 Pa*s und besonders bevorzugt im Bereich von 200 mPa*s bis 10 Pa*s auf, wobei diese Größe mittels Rotationsviskosimetrie bei 110°C gemessen wird. Die Messung kann gemäß DIN EN ISO 3219 bei 30 $s^{-1}$ durchgeführt werden, wobei hierzu beispielsweise zwei 20 mm Platten eingesetzt

werden können.

Die Säurezahl des Polymers mit Polyestereinheiten liegt vorzugsweise im Bereich von 0 bis 20 mg KOH/g, besonders bevorzugt im Bereich von 1 bis 15 mg KOH/g und ganz besonders bevorzugt im Bereich von 6 bis 10 mg KOH/g. Diese Eigenschaft kann durch Titration mit NaOH gemessen werden (vgl. DIN 53402).

Von besonderem Interesse sind insbesondere Polymere mit Polyestereinheiten, die eine Hydroxyzahl im Bereich von 0 bis 200 mg KOH/g, bevorzugt im Bereich von 1 bis 150 mg KOH/g und ganz besonders bevorzugt im Bereich von 10 bis 140 mg KOH/g aufweisen. Diese Eigenschaft wird gemäß ASTM E222 gemessen.

Das Molekulargewicht des Polymers mit Polyestereinheiten ist an sich nicht kritisch, wobei jedoch die zuvor dargelegten Viskositäten eingehalten werden müssen. Je nach Aufbau des Polymers kann dieses ein relativ hohes Molekulargewicht aufweisen. Zweckmäßig kann das Polymer ein Molekulargewicht im Bereich von 1000 g/mol bis 400000 g/mol, bevorzugt 1500 bis 100000 g/mol und ganz besonders bevorzugt 1800 bis 20000 g/mol aufweisen. Diese Größe bezieht sich auf das Gewichtsmittel des Molekulargewichts (Mw), welches mittels Gelpermeationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher einen apparenten Messwert dar.

Darüber hinaus kann das Molekulargewicht der erfindungsgemäß einzusetzenden Polymere aus der Säure- und der Hydroxyzahl bestimmt werden, falls die Komponenten bekannt sind. Dieses Verfahren eignet sich insbesondere bei Polymeren mit kleinem Molekulargewicht. So können bevorzugte Polymere ein aus der Säure- und der Hydroxyzahl bestimmtes Molekulargewicht im Bereich von 1000 bis 30000 g/mol, bevorzugt 1500 bis 15000 g/mol aufweisen.

Das Polymer mit Polyestereinheiten kann beispielsweise einen linearen Aufbau aufweisen. Um die zuvor dargelegten Parameter einzuhalten, haben diese Polymere vielfach ein relativ niedriges Molekulargewicht, das vorzugsweise im Bereich von 1000 g/mol bis 20000 g/mol, besonders bevorzugt 1500 g/mol bis 5000 g/mol liegt. Besonders geeignete lineare Polymere mit Polyestereinheiten sind unter anderem unter der Handelsbezeichnung Dynacoll® von Degussa GmbH erhältlich, wobei insbesondere Dynacoll® 7362 besonders bevorzugt ist. Dynacoll® 7362 ist ein Polyester mit einer Hydroxyzahl im Bereich von 47 bis 54, einem Molekulargewicht von ca. 2000 g/mol, einem Schmelzpunkt von 53°C und einer Viskosität von 0,5 Pa*s, gemessen bei 80°C mittels Rotationsviskosimetrie. Das Molekulargewicht von Dynacoll® 7362 kann insbesondere aus der Säure- und der Hydroxyzahl bestimmt werden.

Von besonderem Interesse sind Verkapselungsmaterialien, die neben mindestens einem Glycerid zusätzlich mindestens ein hyperverzweigtes Polymer umfassen, das einen hydrophilen Kern mit Polyestereinheiten sowie hydrophobe Endgruppen aufweist.

Überraschenderweise wurde gefunden, dass bei Verwendung von hyperverzweigten Polymeren als Bestandteil des Verkapselungsmaterials viele Vorteile erzielt werden können. Beispielsweise können die Verkapselungsverfahren mit deutlich reduzierten Mengen an Lösungsmitteln bzw. komprimierten Gasen durchgeführt werden.

Das hyperverzweigte Polymer kann somit selbst als Lösungsmittel/Dispergiermittel fungieren. Die hierdurch reduzierten Lösungsmittel-/Gaskonzentrationen führen im Vergleich zum Stand der Technik zu sichereren Prozessen, da hyperverzweigte Polymere keine explosionsfähigen Dämpfe wie andere Lösungsmittel des Stands der Technik bilden können.

Bevorzugte Präparate umfassen ein hyperverzweigtes Polymer mit einem hydrophilen Kern. Hydrophil bedeutet, dass der Kern in der Lage ist, einen hohen Anteil an Wasser aufzunehmen. Gemäß einem bevorzugten Aspekt der vorliegenden Erfindung ist der hydrophile Kern in Wasser löslich. Vorzugsweise beträgt die Löslichkeit in Wasser bei 90°C mindestens 10 Massenprozent, besonders bevorzugt mindestens 20 Massenprozent. Diese Größe wird anhand des hyperverzweigten Polymers vor der Hydrophobisierung, d.h. am hydrophilen Kern als solchen, gemessen. Die Messung kann gemäß der sogenannten Kolbenmethode erfolgen, wobei die Wasserlöslichkeit der reinen Substanz gemessen wird.

Bei dieser Methode wird die Substanz (Feststoffe müssen pulverisiert werden) bei einer Temperatur in Wasser aufgelöst, die leicht über der Prüftemperatur liegt. Wenn die Sättigung erreicht ist, wird die Lösung abgekühlt und bei der Prüftemperatur gehalten. Die Lösung wird gerührt, bis das Gleichgewicht erreicht ist. Alternativ kann die Messung unmittelbar bei der Prüftemperatur durchgeführt werden, wenn durch entsprechende Probenahme gesichert ist, dass das Sättigungsgleichgewicht erreicht ist. Dann wird die Konzentration der Prüfsubstanz in der wässrigen Lösung, die keine ungelösten Substanzteilchen enthalten darf, mit einer geeigneten Analysenmethode bestimmt.

Der hydrophile Kern weist vorzugsweise eine vor der Hydrophobisierung gemessene Hydroxyzahl im Bereich von 400 bis 600 mg KOH/g, besonders bevorzugt im Bereich von 450 bis 550 mg KOH/g auf. Diese Eigenschaft wird gemäß ASTM E222 gemessen. Hierbei wird das Polymer mit einer definierten Menge an Essigsäureanhydrid umgesetzt. Nicht umgesetztes Essigsäureanhydrid wird mit Wasser hydrolysiert. Anschließend wird die Mischung mit NaOH titriert. Die Hydroxyzahl ergibt sich aus dem Unterschied zwischen einer Vergleichsprobe und dem für das Polymer gemessenen Wert. Hierbei ist die Anzahl an Säuregruppen des Polymers zu berücksichtigen.

Gemäß einer zweckmäßigen Ausführungsform weist das hyperverzweigte Polymer einen Kern auf, der Polyestereinheiten umfasst. Hyperverzweigte Polymere mit Polyestereinheiten sind insbesondere in EP 0 630 389 dargelegt.

Im Allgemeinen weist der hydrophile Kern eine zentrale Einheit, die von einem Initiatormolekül mit mindestens 2, vorzugsweise mindestens 3 Hydroxygruppen abgeleitet ist, und Wiederholungseinheiten auf, die von Monomeren mit mindestens einer Carboxylgruppe und mindestens 2 Hydroxygruppen abgeleitet sind.

Die Begriffe Initiatormolekül sowie Wiederholungseinheit sind in der Fachwelt weithin bekannt. So können die hyperverzweigten Polymere durch Polykondensation erhalten werden, wobei ausgehend von einem mehrwertigen Alkohol zunächst die Carbonsäuregruppen der Monomere umgesetzt werden. Hierbei werden Estergruppen gebildet. Da die Monomere mindestens 2 Hydroxygruppen umfassen, weist das Makromolekül nach jeder Umsetzung mehr Hydroxygruppen auf, als vor der Umsetzung.

Vorzugsweise ist das Initiatormolekül ein aliphatisches Polyol, mit vorzugsweise 3, 4, 5, 6, 7 oder 8, besonders bevorzugt 3, 4 oder 5 Hydroxygruppen.

Besonders bevorzugt ist das Initiatormolekül ausgewählt aus Ditrimethylolpropan, Ditrimethylolethan, Dipentaerythrit, Pentaerythrit, alkoxliertem Pentaerythrit, Trimethylolethan, Trimethylolpropan, alkoxyliertem Trimethylolpropan, Glycerin, Neopentylalkohol, Dimethylolpropan und/oder 1,3-Dioxan-5,5-dimethanol. Gemäß einem besonderen Aspekt der vorliegenden Erfindung sind die Wiederholungseinheiten von Monomeren mit einer Carboxylgruppe und mindestens 2 Hydroxygruppen abgeleitet. Zu diesen bevorzugten Monomeren gehören insbesondere Dimethylpropionsäure, $\alpha,\alpha$-Bis(hydroxymethyl)buttersäure,
$\alpha,\alpha,\alpha$-Tris(hydroxymethyl)essigsäure,
$\alpha,\alpha$-Bis(hydroxymethyl)valeriansäure,
$\alpha,\alpha$-Bis(hydroxy)propionsäure und/oder
3,5-Dihydroxybenzoesäure.

Ganz besonders bevorzugt ist der hydrophile Kern durch Polymerisation von Dimethylolpropionsäure erhältlich, wobei als Initiatormolekül besonders bevorzugt Ditrimethylolpropan, Trimethylolpropan, ethoxyliertes Pentaerthrit, Pentaerthrit oder Glycerin eingesetzt wird.

Der hydrophile Kern weist vorzugsweise ein Molekulargewicht von mindestens 1500 g/mol, bevorzugt mindestens 2500 g/mol. Diese Größe bezieht sich auf das Gewichtsmittel des Molekulargewichts (Mw), welches mittels Gelpermeationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher einen apparenten Messwert dar.

Vorzugsweise kann der hydrophile Kern eine Glasübergangstemperatur aufweisen, die im Bereich von -40 bis 60 °C, besonders bevorzugt 0 bis 50°C und ganz besonders bevorzugt 10 bis 45°C liegt. Die Glasübergangstemperatur kann durch DSC-Verfahren ermittelt werden, wobei eine Heizrate von 3°C/min verwendet werden kann (DMA Tan $\delta$ peak; Netsch DMA 242 3-point bending 1Hz 3°C/min).

Die Hydrophobisierung der Oberfläche des Polymeren wird im Allgemeinen als letzter Reaktionsschritt durch Umsetzung zumindest eines Teils der freien Hydroxygruppen mit vorzugsweise einer langkettigen Carbonsäure erhalten.

Vorzugsweise beträgt der Funktionalisierungsgrad des hyperverzweigten Kernmoleküls mit hydrophoben Endgruppen, vorzugsweise mit Fettsäure-enthaltenden Bausteinen, mindestens 5%, insbesondere bevorzugt mindestens 30%, besonders bevorzugt mindestens 40%. Gemäß einem weiteren Aspekt der vorliegenden Erfindung liegt der Funktionalisierungsgrad des hyperverzweigten Kernmoleküls mit hydrophoben Endgruppen, vorzugsweise mit Fettsäure-enthaltenden Bausteinen, im Bereich von 30 bis 100%, bevorzugt im Bereich von 35 bis 95%, insbesondere bevorzugt im Bereich von 40 bis 90% und ganz besonders bevorzugt im Bereich von 45 bis 85%.

Der Funktionalisierungsgrad bezieht sich auf den Anteil an Hydroxygruppen, der bei der Hydrophobisierung umgesetzt wird. Dementsprechend kann der Funktionalisierungsgrad bzw. der Veresterungsgrad mit Fettsäuren über die Messung der Hydroxy-Zahl für das hyperverzweigte Kernmolekül vor der Hydrophobisierungsreaktion und nach der Hydrophobierungsreaktion bestimmt werden.

Neben dem hydrophilen Kern weist das hyperverzweigte Polymer hydrophobe Endgruppen auf. In diesem Zusammenhang bedeutet der Begriff hydrophobe Endgruppen, dass mindestens ein Teil der Kettenenden des hyperverzweigten Polymers hydrophobe Gruppen aufweist. Hierbei kann angenommen werden, dass hierdurch eine zumindest teilweise hydrophobisierte Oberfläche erhalten wird.

Der Begriff hydrophob ist an sich in der Fachwelt bekannt, wobei die Gruppen, die zumindest an einem Teil der Enden der hyperverzweigten Polymere vorhanden sind, für sich betrachtet, eine geringe Wasserlöslichkeit aufweisen. Gemäß einem besonderen Aspekt wird die Oberfläche durch Gruppen hydrophobisiert, die von Carbonsäuren mit mindestens 6, bevorzugt mindestens 12 Kohlenstoffatomen abgeleitet sind. Die Carbonsäuren weisen vorzugsweise höchstens 40, besonders höchstens 32 Kohlenstoffatome, besonders bevorzugt höchstens 20 Kohlenstoffatome und ganz besonders bevorzugt höchstens 18 Kohlenstoffatome auf. Hierbei können die Gruppen von gesättigten und/oder ungesättigten Fettsäuren abgeleitet sein. Vorzugsweise beträgt der Anteil der Carbonsäuren mit 12 bis 18 Kohlenstoffatomen mindestens 30 Gew.-%, besonders bevorzugt mindestens 50 Gew.-% und ganz besonders bevorzugt mindestens 60 Gew.-%, bezogen auf das Gewicht der zur Hydrophobisierung eingesetzten Carbonsäuren.

Hierzu gehören insbesondere Fettsäuren, die in Leinsamen, Sojabohnen und/oder Tallöl enthalten sind. Besonders geeignet sind Fettsäuren, die einen geringen Anteil an Doppelbindungen aufweisen, beispielsweise Hexadecensäure, insbesondere Palmitoleinsäure, und Octadecensäure, insbesondere Ölsäure.

Bevorzugte Carbonsäuren weisen hierbei einen Schmelzpunkt von mindestens 35 °C, bevorzugt mindestens 40 °C und besonders bevorzugt mindestens 60 °C auf. Dementsprechend werden bevorzugt lineare, gesättigte Carbonsäuren eingesetzt. Hierzu gehören insbesondere Octansäure, Decansäure, Dodecansäure, Tetradecansäure, Hexadecansäure, Heptadecansäure, Octadecansäure, Eicosansäure, Docosansäure und Tetracosansäure. Besonders bevorzugt sind gesättigte Fettsäuren mit 16 bis 22 Kohlenstoffatomen, insbesondere bevorzugt 16 bis 18 Kohlenstoffatomen.

Von besonderen Interesse sind insbesondere hyperverzweigte Polymere (nach der Hydrophobisierung), die ein Molekulargewicht von mindestens 3000 g/mol, bevorzugt mindestens 6000 g/mol, besonders bevorzugt mindestens 7500 g/mol aufweisen. Vorzugsweise beträgt das Molekulargewicht höchstens 30000 g/mol, besonders bevorzugt höchstens 25000 g/mol. Diese Größe bezieht sich auf das Gewichtsmittel des Molekulargewichts (Mw), welches mittels Gelpermeationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibrierkurve eingesetzt, die unter Verwendung von Polystyrol-Standards erhalten wurde. Diese Größe stellt daher einen apparenten Meßwert dar.

Die Polydispersität Mw/Mn bevorzugter hyperverzweigter Polymere liegt vorzugsweise im Bereich von 1,01 bis 6,0, besonders bevorzugt im Bereich von 1,10 bis 5,0 und ganz besonders bevorzugt im Bereich von 1,2 bis 3,0, wobei das Zahlenmittel des Molekulargewichts (Mn) ebenfalls durch GPC erhalten werden kann.

Das Gewichtsverhältnis von hydrophilem Kern zu den hydrophoben Endgruppen kann vorzugsweise im Bereich von 10:1 bis 1:10, besonders bevorzugt 1:1 bis 1:2,5 liegen. Dieses Verhältnis ergibt sich aus dem Gewichtsmittel des hydrophilen Kerns und dem Gewichtsmittel des hyperverzweigten Polymers.

Der Verzweigungsgrad des hyperverzweigten Polymers liegt im Bereich von 20 bis 70%, vorzugsweise 25 bis 60%. Der Verzweigungsgrad ist abhängig von den zur Herstellung des Polymers, insbesondere des hydrophilen Kerns eingesetzten Komponenten sowie der Reaktionsbedingungen. Der Verzweigungsgrad kann gemäß Frey et al. bestimmt werden, wobei dieses Verfahren in D.Hölter, A.Burgath, H.Frey, Acta Polymer, 1997, 48, 30 und H. Magnusson,E. Malmström, A. Hult, M. Joansson, Polymer 2002, 43, 301 dargelegt ist.

Das hyperverzweigte Polymer weist vorzugsweise eine Schmelztemperatur von mindestens 30°C, besonders bevorzugt mindestens 35°C und ganz besonders bevorzugt mindestens 40°C auf. Gemäß einem besonderen Aspekt der vorliegenden Erfindung kann der Schmelzpunkt des hyperverzweigten Polymers vorzugsweise höchstens 65°C, insbesondere bevorzugt höchstens 60°C, besonders bevorzugt höchstens 57°C und ganz besonders bevorzugt höchstens 55°C betragen. Die Schmelztemperatur kann mittels Differential Scanning Calometry (DSC) erfolgen, z.B. mit dem Apparat Mettler DSC 27 HP und einer Heizrate von 10°C/min.

Die Wasserlöslichkeit des hyperverzweigten Polymers nach der Hydrophobisierung beträgt vorzugsweise höchstens 10 Massenprozent, besonders bevorzugt höchstens 7 Massenprozent und ganz besonders bevorzugt höchstens 5 Massenprozent, gemessen nach der zuvor dargelegten Kolbenmethode bei 40°C.

Vorzugsweise besteht das hyperverzweigte Polymer im Wesentlichen aus Wasserstoff, Sauerstoff und Kohlenstoff. Der Begriff im Wesentlichen bedeutet, dass weitere Elemente bis zu höchstens 10 Gew.-%, besonders bevorzugt höchstens 5 Gew.-% in dem hyperverzweigten Polymer enthalten sind.

Gemäß einem besonderen Aspekt der vorliegenden Erfindung kann das hyperverzweigte Polymer enzymatisch abgebaut werden. Dies kann beispielsweise dadurch erreicht werden, dass der hydrophile Kern und/oder die hydrophobe Hülle enzymatisch abbaubare organische Estergruppen umfasst.

Die Herstellung dieser hyperverzweigten Polymere ist insbesondere in EP 630 389 dargelegt. Im Allgemeinen kann ein Initiatormolekül mit mindestens einer Verbindung umgesetzt werden, die mindestens 2 Hydroxygruppen sowie mindestens eine Carbonsäuregruppe umfasst. Hierdurch wird ein hydrophiler Kern erhalten, der mit mindestens einer hydrophoben Verbindung, beispielsweise einer langkettigen Carbonsäure umgesetzt werden kann.

Im Allgemeinen wird die Reaktion bei einer Temperatur im Bereich von 0°C bis 300°C, vorzugsweise 100°C bis 250°C durchgeführt, wobei die Umsetzung durch bekannte Veresterungskatalysatoren beschleunigt werden kann. Zu diesen Katalysatoren gehören beispielsweise Lewis- und Brønstedtsäuren, insbesondere p-Toluolsulfonsäure, Methansulfonsäure, Trifluoressigsäure, $BF_3$, $AlCl_3$ und $SnCl_4$; Titanverbindungen, insbesondere Tetrabutyltitanat; Zink- und/oder Zinnpulver.

Vorzugsweise wird bei der Veresterung freigesetztes Wasser aus der Reaktionsmischung entfernt.

Der Anteil an Polymer mit Polyestereinheiten, bezogen auf das Gewicht des Präparats liegt vorzugsweise im Bereich von 1 bis 98,5 Gew.-%, besonders bevorzugt im Bereich von10 bis 90 Gew.-% und ganz besonders bevorzugt im Bereich von 20 bis 80 Gew.-%.

Das Gewichtsverhältnis von Polymer mit Polyestereinheiten zu Glycerid mit einem Schmelzpunkt von mindestens 35°C ist an sich nicht kritisch. Überraschend kann jedoch der Anteil an Naturstoff durch einen hohen Anteil an

Polymer erhöht werden, so dass der Beladungsgrad durch diese Maßnahme überraschend gesteigert werden kann. Andererseits kann die Lagerstabilität sowie die enzymatische Abbaubarkeit durch die Verwendung eines hohen Anteils an Glycerid verbessert werden, wobei dieser Befund überraschend ist. Von besonderem Interesse sind daher Präparate, die sich durch ein Gewichtsverhältnis von Polymer mit Polyestereinheiten zu Glycerid auszeichnen, das vorzugsweise im Bereich von 20:1 bis 1:20, besonders bevorzugt 10:1 bis 1:10 und ganz besonders bevorzugt 5:1 bis 1:5 liegt.

Neben einem Verkapselungsmaterial umfassen die erfindungsgemäßen Präparate mindestens einen bioaktiven Naturstoff. Hierbei wird der bioaktive Naturstoff vorzugsweise durch eine nicht kovalente Weise mit dem Verkapselungsmaterial verbunden. Dies kann beispielsweise durch ionische oder polare Wechselwirkungen oder durch Vander-Waals Kräfte erfolgen.

Aufgrund der Wechselwirkung von Verkapselungsmaterial und bioaktiven Naturstoff kann sich das Präparat der vorliegenden Erfindung von einer konventionellen Mischung dieser Komponenten unterscheiden.

Diese Wechselwirkung kann auf bekannte Weise gemessen werden. Je nach Naturstoff sind hierfür vielfach spektroskopische Methoden geeignet. Beispielsweise können teilweise Verschiebungen im Infrarotspektrum beobachtet werden.

Des Weiteren können die erfindungsgemäßen Präparate gegenüber einer konventionellen Mischung eine verzögerte Freisetzung des bioaktiven Naturstoffs in ein Medium zeigen, welches von dem bioaktiven Naturstoff des Präparats verschieden ist. Die verzögerte Freisetzung kann gemäß der von Smirnova, I.; Suttiruengwong, S.; Arlt, W. "Feasibility study of hydrophilic and hydrophobic silica aerogels as drug delivery systems"; Journal of Non-Crystalline Solids (2004) 54-60, beschriebenen Verfahren gemessen werden.

Im Allgemeinen beträgt der Zeitunterschied, um eine identische Konzentration des bioaktiven Naturstoffs in dem Medium zu erhalten, in das der Naturstoff freigesetzt wird, mindestens 1 Minute, bevorzugt mindestens 5 Minuten. Hierbei bezieht sich dieser Zeitunterschied auf die Messung von einem Präparat der vorliegenden Erfindung und die Messung einer konventionellen Mischung dieser Komponenten unter identischen Bedingungen einer verzögerten Freisetzung. Verzögerte Freisetzung bedeutet, dass die Bedingungen nicht so gewählt werden, dass das Präparat den bioaktiven Naturstoff möglichst schnell freisetzt. Diese Bedingungen sind dem Fachmann bei Kenntnis dieser Anmeldung geläufig. Die Werte der konventionellen Mischung können auch durch getrennte Zugabe der Komponenten ermittelt werden.

Um eine gesteuerte Freisetzung zu erzielen, liegt das Präparat vorzugsweise verkapselt vor, wobei der Begriff "Verkapselung" in der Fachwelt bekannt ist. Gemäß einem Aspekt kann der bioaktive Naturstoff beispielsweise in einer Hülle eingebettet werden, die das Verkapselungsmaterial umfasst. Gemäß einer bevorzugten Ausführungsform kann auch eine Matrixverkapselung vorliegen, wobei das Verkapselungsmaterial den bioaktiven Naturstoff in einer Matrix schützt. Vorzugsweise ist der bioaktive Naturstoff dabei in dem Verkapselungsmaterial homogen dispergiert. Besonders bevorzugt bildet die Matrix zusammen mit dem bioaktiven Naturstoff eine einheitliche Phase, so dass der bioaktive Naturstoff in dem Verkapselungsmaterial gelöst vorliegt. Entsprechend diesen Ausführungen kann der Schutz des bioaktiven Naturstoffs durch eine Matrixverkapselung und/oder eine Kern-Hülle-Verkapselung erfolgen. Zweckmäßig können die erfindungsgemäßen Präparate in Form von Mikrokapseln oder Mikropartikeln vorliegen.

Unter dem Begriff "Mikrokapseln" werden erfindungsgemäß Partikel und Aggregate verstanden, die einen inneren Raum oder Kern enthalten, der mit einem festen, gelierten, flüssigen oder gasförmigen Medium gefüllt ist und von einer kontinuierlichen Hülle aus Verkapselungsmaterial umschlossen sind. Diese Teilchen besitzen vorzugsweise kleine Abmessungen.

Zusätzlich können die mikroskopisch kleinen Kapseln einen oder mehrere Kerne im kontinuierlichen Verkapselungsmaterial, bestehend aus einer oder mehreren Lagen, verteilt enthalten. Die Verteilung des einzuhüllenden Materials kann soweit gehen, dass eine homogene Mischung aus Hüll- und Kernmaterial entsteht, was als Matrix bezeichnet wird. Matrixsysteme sind auch als Mikropartikel bekannt.

Entsprechend den zuvor dargelegten Ausführungen kann das Präparat der vorliegenden Erfindung partikelförmig vorliegen. Hierbei weisen diese Partikel vorzugsweise eine Größe im Bereich von 1 bis 1000 $\mu$m, besonders bevorzugt 10 bis 500 $\mu$m auf.

Die Form der Partikel ist an sich unkritisch, wobei die Partikel jedoch vorzugsweise eine sphärische Form aufweisen.

Der Begriff sphärisch bezeichnet im Rahmen der vorliegenden Erfindung, dass die Partikel vorzugsweise eine kugelförmige Gestalt aufweisen, wobei dem Fachmann offensichtlich ist, dass aufgrund der Herstellungsmethoden auch Partikel mit anderer Gestalt enthalten sein können, oder dass die Form der Partikel von der idealen Kugelgestalt abweichen kann.

Dementsprechend bedeutet der Begriff sphärisch, dass das Verhältnis von der größten Ausdehnung der Partikel zur geringsten Ausdehnung maximal 4, vorzugsweise maximal 2 beträgt, wobei diese Ausdehnungen jeweils durch den Schwerpunkt der Partikel gemessen werden. Vorzugsweise sind mindestens 70%, besonders bevorzugt mindestens 90 %, bezogen auf die Zahl der Partikel, sphärisch.

Die Partikelgröße kann auf allgemein bekannte Weise bestimmt werden. Hierzu können beispielsweise mikroskopische Aufnahmen verwendet werden, die visuell und/oder mit Hilfe von Computern ausgewertet werden können.

Des Weiteren weisen bevorzugte Mikropartikel eine besonders enge Partikelgrößenverteilung auf. So liegen bevorzugt mindestens 80 Gew.-% der Partikel innerhalb eines Größenbereichs von 1 μm bis 200 μm, vorzugsweise 1 μm bis 100 μm, besonders bevorzugt 1 μm bis 50 μm.

Gemäß einem besonderen Aspekt der vorliegenden Erfindung weisen vorzugsweise 90% der Partikel eine Größe im Bereich von 1 bis 1000 μm, insbesondere bevorzugt 3 bis 800 μm, besonders bevorzugt 7 bis 700 μm und ganz besonders bevorzugt 10 bis 500 μm auf.

Bevorzugte Präparate gemäß der vorliegenden Erfindung zeigen eine gut steuerbare enzymatische Abbaubarkeit. Von besonderem Interesse sind Präparate, die sich inner halb von drei Tagen oder weniger, bevorzugt 2 Tagen oder weniger und besonders bevorzugt von einem Tag oder weniger abgebaut werden können. Vorzugsweise werden mindestens 20 Gew.-%, besonders bevorzugt mindestens 50 Gew.-% des im Präparat enthaltenen Naturstoffs bei Kontakt mit einem Enzym innerhalb von höchstens drei Tagen, bevorzugt innerhalb von höchstens zwei Tagen und ganz besonders bevorzugt innerhalb von 24 Stunden freigesetzt. Hierbei werden Präparate mit einem geeigneten Enzym, insbesondere einer Lipase, beispielsweise Lipomod® 34P (Biocatalyst Lmt., UK) abgebaut. Beispielsweise können Präparate mit einem Beladungsgrad von 1 bis 20 Gew.-% untersucht werden, wobei vorzugsweise 1 Gew.-% mit Wirkstoff beladene Polymerpartikel in 50 ml Phosphatpuffer (pH 5.01) oder in 50 ml Lösung des Enzyms Lipomod® 34P (Biocatalyst Lmt., UK) in dem gleichen Puffer (mit einer Konzentration des Lipomod® 34P von 0.5 mg/ml) suspendiert werden können. Die Proben können in einem Wasserbad bei 37°C ohne Durchmischung gehalten werden. In regelmäßigen Zeitabständen, beispielsweise 5 Stunden, können Proben von ca. 5 ml entnommen werden, wobei die Konzentration des Wirkstoffs mit geeigneten Verfahren, beispielsweise iodometrische Titration mit Metrohm Titroprocessor 686, analysiert werden kann. Hierbei wird die Freisetzung einer Vergleichsprobe, die kein Enzym umfasst, berücksichtigt, um Probleme der Lagerstabilität bei den gewählten Messbedingungen auszuschließen, so dass sich der zuvor angegebene Wert auf die Differenz zwischen gemessener Probe und Vergleichsprobe ergibt. Die enzymatische Abbaubarkeit des Präparats ergibt sich insbesondere durch die Abbaubarkeit des Verkapselungsmaterials. Bevorzugt sind die Polymere mit Polyestergruppen und die Glyceride enzymatisch abbaubar. Der enzymatische Abbau des Glycerids kann analog der zuvor dargelegten Vorschrift erfolgen, wobei jedoch die Konzentrationszunahme der Fettsäure bestimmt wird. Ähnliches gilt für den Abbau des Polymers, wobei hier die Zunahme der Konzentration an Monomeren, die zur Bildung von Polyester geeignet sind, gemessen werden kann.

Die Präparate der vorliegenden Erfindung können eine hervorragende Scherstabilität zeigen, die vielfach über die Wahl des Verkapselungsmaterials und die Verfahrensbedingungen bei der Präparatherstellung beeinflusst werden kann. Vorzugsweise zeigen die Präparate eine Scherstabilität von 1 Minute oder länger, bevorzugt 5 Minuten oder länger, wobei diese Stabilität bei einer Belastung bestimmt wird, die der eines ULTRA-TURRAX® Rührers bei 15000 Umdrehungen pro Minute, bevorzugt 20000 Umdrehungen pro Minute entspricht. Im Allgemeinen wird hierbei eine Dispersion der Partikel, beispielsweise in einem Pharmaöl (Paraffin Oil WINOG 100 Pharma von Univar GmbH) hergestellt, wobei die Dispersion beispielsweise 10 Gew.-% Präparat enthalten kann. Die Partikel werden vor und nach der Stabilitätsmessung, die beispielsweise durch einen ULTRA-TURRAX® Rührers bei 15000 Umdrehungen pro Minute, bevorzugt 20000 Umdrehungen pro Minute erfolgen kann, mikroskopisch untersucht, wobei die Partikelform, Größe und Verteilung beurteilt wird. Eine Scherstabilität bei den zuvor genannten Bedingungen ist gegeben, falls keine wesentlichen Veränderungen beobachtet werden können.

Die Lagerstabilität der erfindungsgemäßen Präparate ist vielfach ebenfalls überraschend hoch, wobei diese von der Art und Zusammensetzung des Medium, falls die Präparate in Form von Dispersionen oder Emulsionen gelagert werden, und/oder den Lagertemperaturen abhängig ist. Bei bevorzugten Lagerbedingungen können bevorzugte Präparate über einen langen Zeitraum, beispielsweise 10 Tage oder länger, vorzugsweise 30 Tage oder länger und besonders bevorzugt 90 Tage oder länger gelagert werden. Diese Größe kann durch die Freisetzung von Wirkstoff in ein Medium oder den Abbau des bioaktiven Naturstoffs gemessen werden. Hierbei beziehen sich diese Angaben auf den Zeitraum bis zu dem höchstens 10 % des bioaktiven Naturstoffs in ein Medium freigesetzt wurden, in dem das Präparat gelagert werden kann bzw. die Zeit bis zu der höchstens 10 % des bioaktiven Naturstoffs, beispielsweise durch Oxidation abgebaut wurden.

Der Begriff "bioaktiver Naturstoff" umfasst Stoffe, die eine Wirkung auf biologische Systeme aufweisen und aus natürlichen Quellen, insbesondere aus Pflanzen oder Tieren gewonnen werden können.

Vorzugsweise weist der bioaktive Naturstoff eine Molmasse im Bereich von 50 g/mol bis 100000 g/mol, besonders bevorzugt 100 g/mol bis 5000 g/mol und ganz besonders bevorzugt 160 g/mol bis 1500 g/mol auf.

Der bioaktive Naturstoff kann aus einem weiten Gebiet gewählt werden, wobei insbesondere Naturstoffextrakte, insbesondere Phytoextrakte besonders bevorzugt sind. Naturstoffextrakte sind Stoffe bzw. Stoffgemische, die durch Extraktion von Naturstoffen erhalten werden können, wobei Phytoextrakte aus Pflanzen gewonnen werden. Naturstoffextrakte werden üblich keiner chemischen Umwandlung unterzogen, sondern lediglich durch physikalische Verfahren, unter anderem Extraktion, Destillation und Fällungsmethoden aus den Naturstoffen gewonnen. Daher sind derartige Stoffe in der kosmetischen und pharmazeutischen Industrie aufgrund einer hohen Konsumentenakzeptanz sehr begehrt.

Zu den bevorzugten Naturstoffextrakten gehören Zusammensetzungen, die durch Extraktion aus Fruchtbestandteilen (Kerne, Schale, Säfte) wie z.B. von Ananas, Apfel, Banane, Birne, Erdbeere, Grapefruit, Pfirsich, Aprikose, Marille, Granatapfel, Preiselbeere, Moosbeere (Cranberry), Kirsche, Himbeere, Johannisbeere, Kaffee , Mango, Orangen, Passionsfrucht, Sauerkirsche, Weintrauben, Quitte, Soja, Oliven, Kakao, Nuss oder Schlehe aber auch aus Pflanzenbestandteilen (Blätter, Hölzer, Wurzeln) wie z.B. aus Vanille, Kamille, Kaffee, Tee, Eiche, Weihrauch oder Gewürzen oder aus Produkten der Lebensmittelindustrie wie z.B. Rum, Bier, Cognac, Tequila, Weinbrand, Whisky, Kaffeeöl und Malz gewonnen werden. Diese Extrakte können vielfach kommerziell erhalten werden. Hierzu gehören insbesondere Cocoa Absolute 14620, Cocoa LC 10167, Cocoa P 11197, Cocoa U88; alle erhältlich von Degussa GmbH. Natürliche Extrakte sind hierbei Extrakte, die aus natürlichen Quellen erhalten werden können oder Eigenschaften aufweisen, die diesen Extrakten ähnlich sind.

Zu den bevorzugten bioaktiven Naturstoffen gehören des Weiteren insbesondere Geschmacksstoffe, Aromastoffe, natürliche Extrakte, Enzym modifizierte Nahrungsmittelzusätze, natürliche Wachse, Proteine, Peptide, Vitamine und Vitaminvorstufen, Fette und Fettsäuren, Aminosäuren und Aminosäurevorstufen, beispielsweise Kreatin, Zucker und Zuckerderivate, Nukleotide, Nukleinsäuren sowie Vorstufen und Derivate derselben, beispielsweise DNA- und RNA-Oligomere, Arzneimittel, Enzyme und Coenzyme.

Von besonderem Interesse sind unter anderem bioaktive Naturstoffe, die mindestens eine Verbindung ausgesucht aus der Gruppe bestehend Tocopherol und Derivate, Ascorbinsäure und Derivate, Desoxyribonucleinsäure, Retinol und Derivate, Alpha-Liponsäure, Niacinamid, Ubichinon, Bisabolol, Allantoin, Phytantriol, Panthenol, AHA-Säuren, Aminosäuren, Hyaluronsäure, Polyglutaminsäure, Beta-Glucane, Creatin und Creatinderivate, Guanidin und Guanidinderivate, Ceramide, Sphingolipide, Phytosphingosin und Phytosphingosinderivate, Sphingosin und Sphingosinderivate, Sphinganin und Sphinganinderivate, Pseudo-Ceramiden, essentielle Ölen, Peptiden, Proteinen, Proteinhydrolysaten, Pflanzenextrakten und Vitaminkomplexen umfassen. Derivate der zuvor dargelegten Stoffe sind insbesondere Verbindungen, die eine im Wesentlichen ähnliche Wirkung aufweisen, wie der jeweilige Stoff an sich. Vielfach können Organismen die Derivate in den entsprechenden Stoff überführen, so dass durch diese Derivate eine ähnliche Wirkung erzielt wird, wie durch Gabe des jeweiligen Stoffes.

Zu den bevorzugten Coenzymen gehört insbesondere Coenzym Q10 (2,3-Dimethoxy-5-methyl-6-polyprenyl-1-benzochinon)

Zu den Vitaminen gehören insbesondere Vitamin A, Vitamine des B-Komplexes, beispielsweise Vitamin B1, Vitamin B2, Vitamin B3 (Folsäure) und Vitamin B12, Vitamin C (Ascorbinsäure), Vitamine des D-Komplexes, insbesondere 7-Dehydrocholesterol, Lumisterol, Calciferol, Ergocalciferol, Cholecalciferol, 22,23-Dihydroergocalciferol und Sitocalciferol, und Vitamin E (Tocopherol) und Vitamin K (Phyllochinon, Menachinon).

Zu den bevorzugten Aminosäuren gehören insbesondere DL-Methionin, L-Lysin, L-Threonin, L-Tryptophan, L-Theanin und L-Leucin.

Zu den Aromastoffen gehören insbesondere Alkane, Alkene, Ketone, Aldehyde, schwefelhaltige Verbindungen, Heterocyclen, Carbonsäureester, Alkohole und/oder natürliche Extrakte, beispielsweise Limonen oder Linalool.

Beispiele für Fette sind insbesondere Öle, die sich von tierischem oder pflanzlichem Material ableiten, und die erfindungsgemäß verwendet werden können, sind Olivenöl, Palmöl, Rapsöl, Flachsöl und Öle der Samen bzw. Kerne von z.B. Sonnenblume, Apfel, Birne, Zitrusfrüchten, wie Mandarine, Orange, Grapefruit oder Zitrone, Melone, Kürbis, Himbeere, Brombeere, Holunder, Johannisbeere, Granatapfel, Weizen- und Reiskeimen sowie Baumwollsaaten, Soja- und Palmkernen. Öle, die von tierischem Talg abgeleitet sind, insbesondere, Rindertalg, Knochenöl, Fischöle, wobei insbesondere PUFA-haltige Öle (Poly Unsaturated Fatty Acid) bevorzugt sind, die einen hohen Anteil an Omega-3-Fettsäuren und Omega-6-Fettsäuren enthalten.

Besonders bevorzugte Öle sind insbesondere Kernöle. Kernöle lassen sich insbesondere aus den Kernen bzw. Samen von Pflanzen extrahieren, wobei hierfür insbesondere Rückstände aus der Verarbeitung von Früchten und Beeren und besonders bevorzugt aus der Saftherstellung geeignet sind. Insbesondere Rückstände von der Verarbeitung von z.B. Apfel, Pfirsich, Birne, Zitrusfrüchten, wie Mandarine, Orange, Grapefruit oder Zitrone, Melone, Kürbis, Himbeere, Brombeere, Holunder, Kirsche, Hagebutte, Aprikose, Erdbeere, Johannisbeere und Granatapfel kommen als Ausgangsmaterialien in Frage. Verfahren zur Gewinnung von bevorzugten Ölen aus pflanzlichen Bestandteilen durch Extraktion sind insbesondere aus der Druckschrift DE A 10 2005 037 209 bekannt. Kernöle weisen vielfach einen hohen Gehalt an Triglyceriden von ungesättigten Fettsäuren auf. So beträgt der Anteil an Ölsäure und/oder Linolsäure vielfach 50 bis 90 Gew.-%. Darüber hinaus sind Kernöle reich an Vitaminen, beispielsweise Vitamin A und/oder Mineralien.

Darüber hinaus können insbesondere Carotinoide, Flavonoide, wie Resveratrol und Xantuhomol, Isoflavonoide, Terpene, Phytosterole, wie Beta-sitosterol, Glycoconjugate, wie Aloeresin, Aloenin, Triterpenoide, wie beispielsweise 11-Keto-β-boswelliasäure oder Acetyl-11-Keto-β-boswelliasäure die aus Weihrauch gewonnen werden können, Polyphenole, insbesondere Lupeol, Squalen, Hydroxytyrosol, Tocopherole und Vanilin als bioaktiver Naturstoff eingesetzt werden.

Darüber hinaus sind natürliche Wachse Beispiele für bevorzugt einsetzbare Naturstoffe. Diese Wachse sind meist von pflanzlicher oder tierischer Herkunft, womit sie typische Naturprodukte darstellen. Eine strenge Abgrenzung

der pflanzlichen und tierischen Wachse aufgrund der an ihrem Aufbau beteiligten Fettsäuren und Fettalkoholen kann nicht vorgenommen werden. Es ist allerdings unstrittig, dass es sich bei Montansäure, Palmitinsäure und Stearinsäure um typische an diesem Naturwachsen beteiligte Fettsäuren handelt. Auf der Seite der Alkohole sind hier vor allem Cetylalkohol und Cerylalkohol zu nennen. Echte Naturwachse pflanzlicher Herkunft sind bspw. Palmblätterwachse, wie Carnaubawachs, Palmwachs, Raffiawachs, Ouricourywachs, Gräserwachse, wie z. B. Candellilawachs, Espartowachs, Fiberwachs und Zuckerrohrwachs; Beeren- und Fruchtwachse sind bspw. Apfelwachs, Birnenwachs, Quittenwachs, Japanwachs, Bayberry-Wachs und Myrthenwachs. Das bekannteste Beispiel eines echten Naturwachses tierischer Herkunft ist das Bienenwachs, welches zur Hauptsache aus Palmitinsäuremiricylester, also einer mit Miricylalkohol veresterten Palmitinsäure, besteht. Bekannt sind auch chinesische Insektenwachse, Schellackwachs und Wollwachse, wie sie bspw. aus Schafwolle gewonnen werden können. Verfahren zur Gewinnung von bevorzugten Wachsen aus pflanzlichen Bestandteilen durch Extraktion sind insbesondere aus der Druckschrift DE A 10 2005 037 210 bekannt.

Die zuvor dargelegten bioaktiven Naturstoffe können einzeln oder als Mischung von zwei, drei oder mehr eingesetzt werden. Hierbei können die Mischungen Naturstoffe der gleichen Klasse oder von unterschiedlichen Klassen umfassen. So kann beispielsweise eine Kombination eine Mischung umfassen, die ein oder mehrere Fruchtwachse und/oder ein oder mehrere Kernöle aufweist.

Die erfindungsgemäßen Präparate können einen überraschend hohen Anteil an bioaktiven Naturstoffen aufweisen. Gemäß einem besonderen Aspekt der vorliegenden Erfindung kann das Gewichtsverhältnis von Verkapselungsmaterial zu Naturstoff vorzugsweise im Bereich von 40:1 bis 0,5:1, besonders bevorzugt im Bereich von 20:1 bis 2:1 liegen. Der Beladungsgrad kann vorzugsweise in einem Bereich von 1% bis 95%, besonders bevorzugt 5% bis 90%, weiter bevorzugt 10 bis 60% und ganz besonders 10 bis 30% liegen, wobei der Beladungsgrad durch den Gewichtsanteil des bioaktiven Naturstoffs am Gesamtgewicht des Präparats gegeben ist.

Der bioaktive Naturstoff kann aus dem erfindungsgemäßen Präparat an sich auf jede gewünschte Weise freigesetzt werden. Beispielsweise kann ein enzymatischer Abbau erfolgen, um die freizusetzende Substanz freizusetzen. Hierbei kann der Freisetzungszeitraum durch die Abbaurate gesteuert werden.

Weiterhin kann die Freisetzung über eine Änderung des pH-Wertes, Temperatur, pH, Strahlungsfrequenz und Art des Mediums gezielt gesteuert werden.

Die Art des Mediums kann beispielsweise über die Zugabe von Lösungsmittel, Tensiden oder Salzen geändert werden. Als Lösungsmittel zur Variation des Mediums können unter anderem Wasser oder Alkohole, wie Ethanol oder Isopropanol eingesetzt werden.

Des Weiteren kann die Freisetzungsrate durch den Anteil an Polymer mit Polyestereinheiten gesteuert werden. Hierbei sind der Funktionalisierungsgrad des hyperverzweigten Polymers oder die Hydroxyzahl des Polymers Parameter, die zur Steuerung der Freisetzung eingesetzt werden können. Je nach Funktionalisierungsgrad des hyperverzweigten Polymers und dem Medium, in das die freizusetzende Substanz freigesetzt werden soll, können somit die unterschiedlichsten Lösungsmittel zugegeben werden, um eine möglichst retardierte oder eine möglichst schnelle Freisetzung zu erzielen. Soll die Freisetzung des verkapselten Naturstoffs in polaren Medien erfolgen, so ist die Freisetzung umso langsamer, je mehr OH-Gruppen des hyperverzweigten Kernpolyesters mit Fettsäuren verestert/funktionalisiert wurden. Dieser Effekt kann durch Zugabe von entsprechenden Lösungsmitteln unterstützt werden.

Weiterhin kann die Wirkstofffreisetzung insbesondere über den Anteil an Verkapselungsmaterial, das den bioaktiven Naturstoff schützt, und/oder den Funktionalisierungsgrad / Hydrophobisierungsgrad bzw. die Hydroxyzahl des Polymers mit Polyestereinheiten gesteuert werden.

Der Freisetzungszeitraum ist umso größer, je höher der Anteil an Verkapselungsmaterial im Präparat ist. Überraschenderweise wurde gefunden, dass bei Verwendung von hyperverzweigten Polymeren die Konzentration in der Ausgangsmischung auch über die im Stand der Technik üblichen Polymerkonzentrationen von 10 Massenprozent bis zu einer Polymerkonzentration von 70 Massenprozent erhöht werden kann. Die letztendlich gewählte Konzentration an Glycerid und an Polymer entscheidet zusammen mit der Temperaturführung bzw. der Änderung von pH-Wert oder Lösungskraft des Lösungsmittels über den Anteil an Verkapselungsmaterial und damit über den Freisetzungszeitraum.

Zur Herstellung der erfindungsgemäßen Präparate können die niedermolekularen Verbindungen sowie das Verkapselungsmaterial miteinander kombiniert werden. Hierzu sind verschiedene Methoden, insbesondere RESS-, GAS , PCA-, SEDS- und/oder PGSS-Verfahren geeignet. Derartige Verfahren sind an sich weithin bekannt und beispielsweise in Gamse et al., Chemie Ingenieur Technik 77 (2005), No. 6, Seiten 669 bis 679 beschrieben.

Von besonderem Interesse sind insbesondere Verfahren zur Herstellung der erfindungsgemäßen Präparate umfassend die Schritte

a) Herstellen einer Schmelze umfassend mindestens ein Polymer mit Polyestereinheiten, mindestens ein Glycerid mit einem Schmelzpunkt von mindestens 35°C und mindestens einen bioaktiven Naturstoff,

b) Einleiten der Schmelze in eine zweite flüssige Phase, in der das Verkapselungsmaterial schwer löslich ist, wobei die zweite flüssige Phase eine Verfestigungstemperatur unterhalb der Verfestigungstemperatur des Verkapselungsmaterials aufweist,

c) Dispergieren der Schmelze in der zweiten flüssigen Phase bei einer Temperatur, die größer oder gleich der Verfestigungstemperatur des Verkapselungsmaterials ist und

d) Verfestigen der in der zweiten flüssigen Phase dispergierten Schmelze.

Das zuvor dargelegte Verfahren zur Herstellung der erfindungsgemäßen Präparate kann besonders einfach und wirtschaftlich durchgeführt werden, wobei insbesondere auf die Verwendung von gesundheitsbedenklichen Lösungsmitteln verzichtet werden kann. Darüber hinaus lassen sich eine Reihe weiterer Vorteile erzielen. Hierzu gehört unter anderem, dass durch das zuvor dargelegte Verfahren die Herstellung von Präparaten, insbesondere von Mikropartikeln ohne hohen apparativen Aufwand und ohne die Verwendung von gesundheitlich bedenklichen Lösungsmitteln gelingt. Durch das erfindungsgemäße Verfahren können insbesondere besonders einheitliche Mikropartikel mit einer vorgegebenen Partikelgröße und Partikelgrößenverteilung erhalten werden. Hierbei ist das Verfahren besonders flexibel. So können mit einer Anlage sowohl kleine als auch große Partikel mit einer jeweils relativ engen Partikelgrößenverteilung hergestellt werden. Ferner können Mikropartikel gebildet werden, die pulverförmige und/oder flüssige Naturstoffe aufweisen. Des Weiteren können die bioaktiven Naturstoffe auch im Verkapselungsmaterial löslich sein oder ein Lösungsmittel für das Verkapselungsmaterial darstellen. Darüber hinaus kann das Verfahren bei relativ geringen Temperaturen durchgeführt werden, so dass temperatursensitive Naturstoffe verkapselt werden können. Darüber hinaus kann das Verfahren der vorliegenden Erfindung mit einem hohen Durchsatz durchgeführt werden, so dass in kurzer Zeit große Mengen an Partikeln gebildet werden können. Durch das vorliegende Verfahren können die Partikel kontinuierlich gebildet werden. Die Anlagen zur Durchführung des erfindungsgemäßen Verfahrens erfordern hierbei im Allgemeinen nur sehr geringe Investitions- und Betriebskosten, da die Anlagen auch bei Normaldruck betrieben werden können und vielfach keine explosiven Gemische gebildet werden, wobei im Allgemeinen auf den Einsatz von gesundheitlich bedenklichen Stoffen verzichtet werden kann. Beim Betrieb benötigen die Anlagen im Allgemeinen nur geringe Mengen an Energie. Darüber hinaus weisen die Anlagen vielfach eine geringe Komplexität auf, so dass die Wartungskosten niedrig sind und die Anlagen einfach und sicher gesteuert werden können.

Gemäß einem bevorzugten Verfahren zur Herstellung der erfindungsgemäßen Präparate wird eine Schmelze hergestellt, die mindestens ein Polymer mit Polyestereinheiten, mindestens ein Glycerid mit einem Schmelzpunkt von mindestens 35°C und mindestens einen bioaktiven Naturstoff umfasst. Der bioaktive Naturstoff wird vorzugsweise fein in der Schmelze, die das Glycerid und das Polymer mit Polyestereinheiten umfasst, verteilt. Hierzu können bekannte Vorrichtungen, wie zum Beispiel Rührwerke, die einen Rührkessel mit Propeller-, Scheiben-, Zahnscheiben-, Anker-, Wendel-, Blatt-, Schaufel-, Schrägblatt-, Kreuzblatt-, Schrauben-, MIG®-, INTERMIG®-, ULTRA-TURRAX®, Schnekken-, Band-, Finger-, Korb-, Impeller-Rührer umfassen, sowie Dispergatoren und Homogenisatoren, die unter anderem mit Ultraschall arbeiten können, eingesetzt werden. Die Vorrichtungen können im Allgemeinen mindestens eine Welle aufweisen, an der wiederum vorzugsweise 1 bis 5 Rührelemente angebracht sein können.

Hierbei kann beispielsweise eine Lösung, eine Suspension oder eine Dispersion entstehen, wobei die Teilchengröße der verteilt vorliegenden Phase vorzugsweise höchstens 5000 μm, besonders bevorzugt höchstens 1000 μm beträgt, falls der bioaktive Naturstoff partikelförmig vorliegt.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 20000 Umdrehungen pro Minute liegen.

Die Temperatur bei der die Schmelze hergestellt wird, kann ebenfalls in einem weiten Bereich liegen, wobei diese unter anderem von der Verfestigungstemperatur des Polymers mit Polyestereinheiten bzw. dem Glycerid abhängig ist. Vorzugsweise liegt die Temperatur im Bereich von 40°C bis 200°C, besonders bevorzugt im Bereich von 45°C bis 100°C. Der beim Herstellen der Schmelze eingesetzte Druck ist ebenfalls unkritisch, wobei dieser vielfach von der Art des bioaktiven Wirkstoffs und der Verfestigungstemperatur des Verkapselungsmaterials abhängig ist. Beispielsweise kann der Druck im Bereich von 0,1 mbar bis 200bar, bevorzugt im Bereich von 10 mbar bis 100 bar gewählt werden.

Gemäß einem besonderen Aspekt der vorliegenden Erfindung wird der Schmelze vorzugsweise kein Lösungsmittel, insbesondere kein organisches Lösungsmittel zugegeben, wobei besonders bevorzugte Schmelzen kein Lösungsmittel umfassen. Als Lösungsmittel wird hierbei eine Substanz verstanden, in der das Verkapselungsmaterial löslich ist und die während des Herstellungsprozesses abgetrennt werden muss, da diese Verbindung nicht in den Präparaten, insbesondere den Mikropartikeln enthalten sein sollte. In diesem Zusammenhang ist festzuhalten, dass viele der zuvor dargelegten Naturstoffen Eigenschaften eines Lösungsmittels haben können. Diese Substanzen sind jedoch ein gewollter Bestandteil der Mikrokapseln, so dass diese Verbindungen in Rahmen der vorliegenden Erfindung kein Lösungsmittel darstellen. Dementsprechend ist die Verwendung von Lösungsmittel zur Durchführung des Verfahrens nicht notwendig. Andererseits werden einige Naturstoffe in gelöster Form geliefert, wobei die hierfür verwendeten Lösungsmittel im Allgemeinen für Verwendung des Naturstoffs nicht kritisch sind, so dass diese beispielsweise gesundheitlich unbedenklich sind. Derartige Hilfsstoffe müssen nicht notwendig vor der Herstellung der Schmelze abtrennt werden. Vielmehr können diese Hilfssubstanzen in die Schmelze eingearbeitet werden.

Die zuvor beschriebene Schmelze wird erfindungsgemäß in eine zweite flüssige Phase überführt, in der das Verkapselungsmaterial schwer löslich ist und die eine Verfestigungstemperatur unterhalb der Verfestigungstemperatur des Verkapselungsmaterials aufweist. Dementsprechend umfasst die zweite flüssige Phase ein oder mehrere Substanzen, die mit dem Verkapselungsmaterial nicht mischbar sind und die als Hauptbestandteil der kontinuierlichen Phase dienen. Da das Verkapselungsmaterial bzw. die Schmelze hydrophob ist, ist dementsprechend die zweite flüssige Phase vorzugsweise hydrophil.

Der Begriff "schwer löslich" bedeutet, dass die Löslichkeit des Verkapselungsmaterials in der zweiten flüssigen Phase möglichst gering sein sollte. Die Löslichkeit ist vielfach von der Temperatur abhängig. Dementsprechend können die Dispergierbedingungen vielfach so gewählt werden, dass ein möglichst geringer Anteil des Verkapselungsmaterials in der zweiten flüssigen Phase gelöst wird. Vorzugsweise weist das Verkapselungsmaterial, insbesondere das Polymer mit Polyestereinheiten und das Glycerid, eine Löslichkeit nach der Kolbenmethode bei der Dispergiertemperatur von höchstens 20 Massenprozent, bevorzugt höchstens 10 Massenprozent in der zweiten flüssigen Phase auf. Gemäß einem besonderen Aspekt kann das Verkapselungsmaterial vorzugsweise eine Löslichkeit nach der Kolbenmethode bei 40°C von höchstens 20 Massenprozent in der zweiten flüssigen Phase aufweisen.

Die zweite flüssige Phase weist eine Verfestigungstemperatur unterhalb der Verfestigungstemperatur des Verkapselungsmaterials auf. Im Allgemeinen ergibt sich diese Temperatur aus der Schmelztemperatur oder der Glasübergangstemperatur des Hauptbestandteils der zweiten flüssigen Phase, wobei Gefrierpunktserniedrigungen durch Hilfs- oder Zusatzstoffe bzw. durch die Verwendung von Stoffgemischen auftreten können. Diese Größe kann aus DSC-Messungen erhalten werden, wobei die Schmelzpunkte bzw. Gefrierpunkte der üblichen Hauptbestandteile der zweiten flüssigen Phase in Nachschlagewerken aufgeführt sind.

Zu den hydrophilen Substanzen, die als Hauptbestandteil in der zweiten flüssigen Phase enthalten sein können, gehören insbesondere Wasser und Alkohole mit 1 bis 7, vorzugsweise 1 bis 4 Kohlenstoffatomen, insbesondere Methanol, Ethanol, Propanol und/oder Butanol, wobei Wasser besonders bevorzugt ist.

Die zweite flüssige Phase kann neben dem Hauptbestandteil zusätzliche Hilfsstoffe, insbesondere Dispergatoren und Stabilisatoren umfassen. Diese Hilfsstoffe sind in der Fachwelt bekannt, wobei Dispergatoren einer Aggregation der Partikel entgegenwirken. Hierzu gehören insbesondere Emulgatoren, Schutzkolloide und Tenside, die jeweils entsprechend den eingesetzten Verkapselungsmaterialien, bioaktiven Naturstoffen und dem Hauptbestandteil der zweiten flüssigen Phase ausgewählt werden können. Zu den bevorzugten Tensiden gehören insbesondere anionische Tenside, wie Laurylethersulfat, kationische Tenside und nichtionische Tenside, wie beispielsweise Polyvinylalkohole und ethoxylierte Fettalkohole. Stabilisatoren können für eine Vielzahl von Anwendungen eingesetzt werden, wobei diese Hilfsstoffe einen erwünschten, instabilen Zustand erhalten bzw. stabilisieren. Hierzu gehören insbesondere Absetzverhinderungsmittel, wie Pektine und/oder Karragenan.

Bevorzugt umfasst die zweite flüssige Phase 60 bis 100 Gew.-% an Hauptbestandteil, beispielsweise die zuvor dargelegten hydrophilen Substanzen, wie Wasser oder Alkohole mit bis zu 4 Kohlenstoffatomen. Weiterhin kann die zweite flüssige Phase 0 bis 40 Gew.-% Hilfssubstanzen enthalten, insbesondere 0 bis 20 Gew.-% Emulgatoren und 0 bis 20 Gew.-% Stabilisatoren.

Die in die zweite flüssige Phase eingebrachte Schmelze wird bei einer Temperatur, die größer oder gleich der Verfestigungstemperatur des Verkapselungsmaterials ist, dispergiert.

Die Verfestigungstemperatur des Verkapselungsmaterials bezeichnet im Rahmen der vorliegenden Erfindung die Temperatur bei der das Verkapselungsmaterial fest wird, so dass Partikel bei dieser Temperatur nicht mehr ohne äußere Einwirkungen zu größeren Aggregaten agglomerieren. Je nach Aufbau und Kristallisationseigenschaften kann die Verfestigungstemperatur beispielsweise durch die Glasübergangstemperatur bzw. die Schmelztemperatur des Polymers mit Polyestereinheiten oder des Glycerids gegeben sein, die beispielsweise durch DSC-Verfahren (Differential Scanning Calorimetry; Dynamische Differenzkalorimetrie) bestimmt werden können. Hierbei ist festzuhalten, dass amorphe Polymere im Allgemeinen lediglich eine Glasübergangstemperatur aufweisen, wohingegen kristalline Polymere eine Schmelztemperatur zeigen. Teilkristalline Polymere können sowohl eine Glasübergangstemperatur als auch eine Schmelztemperatur zeigen, wobei in diesem Fall die Temperatur ausschlaggebend ist, bei der die Partikel keine Agglomeration zeigen. Falls die Oberfläche im Wesentlichen kristallin ist, so ist der Schmelzpunkt dieser Bestandteile entscheidend.

Dispergieren bedeutet in diesem Zusammenhang, dass die mindestens einen bioaktiven Naturstoff umfassende Schmelze fein in der kontinuierlichen zweiten flüssigen Phase verteilt wird. Das Dispergieren kann hierbei mit bekannten Geräten und Vorrichtungen, wie zum Beispiel Rührwerken, die einen Rührkessel mit Propeller-, Scheiben-, Zahnscheiben-, Anker-, Wendel-, Blatt-, Schaufel-, Schrägblatt-, Kreuzblatt-, Schrauben-, MIG®-, INTERMIG®-, ULTRA-TURRAX®, Schnecken-, Band-, Finger-, Korb-, Impeller-Rührer umfassen sowie Dispergatoren und Homogenisatoren, die unter anderem mit Ultraschall arbeiten können, durchgeführt werden. Die Vorrichtungen können im Allgemeinen mindestens eine Welle aufweisen, an der wiederum vorzugsweise 1 bis 5 Rührelemente angebracht sein können.

Die Dauer und der Energieeintrag des Dispergierens sind hierbei von der gewünschten Partikelgröße und Partikelgrößenverteilung abhängig. Dementsprechend kann die Dauer des Dispergierens in einem weiten Bereich gewählt werden. Vorzugsweise wird das Dispergieren für eine Zeitdauer im Bereich von 1 Sekunde bis 5 Stunden, besonders bevorzugt im Bereich von 10 Sekunden bis 2 Stunden durchgeführt.

Gemäß einem besonderen Aspekt der vorliegenden Erfindung kann die Newtonzahl beim Dispergieren vorzugsweise im Bereich von 0,1 bis 1000, besonders bevorzugt im Bereich von 0,4 bis 800 liegen.

Die Newtonzahl errechnet sich aus der Formel

$$N_{Po} = P \cdot \rho^{-1} \cdot n^{-3} \cdot d^{-5}$$

wobei

- P die Rührleistung [W] bzw. [$kg^{-}m^{-2} \cdot S^{-3}$],
- d der Durchmesser des Rührers [m],
- ρ die Dichte der Flüssigkeit in dem System [$kg^{-}m^{-3}$]
- und n die Frequenz bzw. die Rotationsgeschwindigkeit [$s^{-1}$] bedeutet.

Entsprechend einer besonderen Ausführungsform des erfindungsgemäßen Verfahrens kann die Reynoldszahl beim Dispergieren vorzugsweise im Bereich von 1 bis $10^7$, besonders bevorzugt im Bereich von 10 bis $10^6$ liegen.

Die Reynoldszahl berechnet sich bei einem gerührten Reaktor entsprechend der folgenden Formel:

$$N_{Re} = n \cdot L^2 \cdot \rho \cdot \mu^{-1}$$

wobei

- n die Frequenz bzw. die Rotationsgeschwindigkeit $s^{-1}$],
- L die charakteristische Länge des Systems [m],
- ρ die Dichte der Flüssigkeit in dem System [$kg \cdot m^{-3}$]
- und μ die dynamische Viskosität der Flüssigkeit in dem System [$kg \cdot m^{-1} s^{-1}$]

bedeutet.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 10000 Umdrehungen pro Minute liegen.

Hierbei ist die eingesetzte Newtonzahl bzw. die Rührgeschwindigkeit von der gewünschten Partikelgröße und Partikelgrößenverteilung abhängig. Je mehr Energie zugeführt wird und je länger dispergiert wird, desto kleinere Partikelgrößen können erzielt werden. Eine enge Partikelgrößenverteilung kann ebenfalls durch eine hohe Dispergierenergie und eine lange Dispergierzeit erzielt werden. Andererseits sind lange Dispergierzeiten und hohe Dispergierenergien vielfach mit zusätzlichen Kosten verbunden.

Die Temperatur bei der die Schmelze in der zweiten flüssigen Phase dispergiert wird, kann ebenfalls in einem weiten Bereich liegen, wobei diese unter anderem von der Verfestigungstemperatur des Verkapselungsmaterials abhängig ist. Vorzugsweise liegt die Temperatur im Bereich von 40°C bis 200°C, besonders bevorzugt im Bereich von 45 bis 100°C. Der beim Dispergieren der Schmelze eingesetzte Druck ist ebenfalls unkritisch, wobei dieser vielfach von der Art des bioaktiven Naturstoffs und der Verfestigungstemperatur des Verkapselungsmaterials abhängig ist. Beispielsweise kann der Druck im Bereich von 10 mbar bis 200 bar, bevorzugt im Bereich von 100 mbar bis 100 bar gewählt werden.

Die Temperatur beim Dispergieren ist größer oder gleich der Verfestigungstemperatur des Verkapselungsmaterials. Vorzugsweise liegt die Dispergiertemperatur 1°C bis 100°C, besonders bevorzugt 5°C bis 70°C und ganz besonders bevorzugt 10 bis 50°C oberhalb der Verfestigungstemperatur des Verkapselungsmaterials.

Das Gewichtsverhältnis von Schmelze zur zweiten flüssigen Phase kann in einem weiten Bereich liegen. Vorzugsweise liegt dieses Verhältnis im Bereich von 1:1 bis 1:200, besonders bevorzugt 1:1,5 bis 1:10.

Beim Dispergieren kann die Zusammensetzung beispielsweise 50 bis 99 Gew.-%, bevorzugt 70 bis 98 Gew.-% zweite flüssige Phase und 1 bis 50 Gew.-%, bevorzugt 2 bis 30 Gew.-% Schmelze umfassen.

Nach dem die Schmelze in der zweiten flüssigen Phase dispergiert vorliegt, wird die dispergierte Schmelze verfestigt. Die Verfestigung kann mit bekannten Methoden, beispielsweise durch Zugabe von Salzen bei einer Temperatur

leicht oberhalb der Verfestigungstemperatur oder durch Abkühlen erfolgen. Vorzugsweise erfolgt das Verfestigen der Schmelze durch Abkühlen der zweiten flüssigen Phase auf eine Temperatur unterhalb der Verfestigungstemperatur des Verkapselungsmaterials.

Die Art der Abkühlung ist unter anderem von der gewünschten Partikelgröße und Partikelgrößenverteilung abhängig. Eine schnelle Abkühlung kann unter anderem zu einer besonders einheitlichen Partikelgrößenverteilung und kleinen Partikeln führen, da eine Aggregation vermieden werden kann. Hierbei ist die Bildung von Agglomeraten bei einem großen Abkühlvolumen geringer.

Des Weiteren können die Partikelgrößenverteilung und die Größe der Partikel über Hilfsmittel, wie beispielsweise Dispergatoren und Emulgatoren, beeinflusst werden. Diese Additive können beispielsweise in die zweite Phase hinzu gegeben werden, wobei eine Additivierung der Oberfläche der gebildeten Partikel erzielt werden kann. Diese Additivierung kann auch eine Aggregation der Mikropartikel während des Trocknens oder beim Lagern vermindern.

Je nach Anwendung kann die so erhaltene Zusammensetzung unmittelbar weiter verarbeitet werden, ohne dass eine Aufreinigung, Aufkonzentration oder Abtrennung vorgenommen wird. Gemäß einer besonderen Ausführungsform kann das vorliegende Verfahren den Schritt des Abtrennens der in der zweiten flüssigen Phase gebildeten Mikropartikel aufweisen. Das Abtrennen kann durch bekannte Verfahren, insbesondere durch Filtrieren, Zentrifugieren, Sedimentation, Magnettrennung, Flotation, Sieben oder Dekantieren erfolgen, wobei die Verfahren einzeln oder in Kombination eingesetzt werden können. Hierbei können die Verbindungen der zweiten flüssigen Phase im Wesentlichen vollständig abgetrennt werden, so dass getrocknete Mikropartikel erhalten werden oder die Partikel können aufkonzentriert werden

Die zum Abtrennen oder Aufkonzentrieren der Mikropartikel verwendbaren Vorrichtungen, nachfolgend auch Separatoren genannt, sind allgemein bekannt. So können unter anderem Zentrifugen, Dekanter, Fliehkraftabscheider, Filter, beispielsweise Schwerkraftfilter, Saugfilter (Vakuumfilter), Druckfilter, Saug-Druck-Filter, Pressfilter, Vakuum-Trommelfilter, Bandfilter, Scheibenfilter, Planfilter, Kammerfilterpresse, Rahmenfilterpresse, Kerzenfilter, Blattfilter, Membranfilterplatte und/oder Siebbandpressen eingesetzt werden.

Die Temperatur beim Abtrennen oder Aufkonzentrieren kann ebenfalls in einem weiten Bereich liegen, wobei diese unter anderem von der Verfestigungstemperatur des Verkapselungsmaterials abhängig ist. Um eine Aggregation der Partikel zu vermeiden, sollte die gewählte Temperatur unterhalb der Verfestigungstemperatur des Verkapselungsmaterials liegen. Vorzugsweise liegt die Temperatur im Bereich von -20°C bis 80°C, besonders bevorzugt im Bereich von -10°C bis 40°C. Der beim Abtrennen oder Aufkonzentrieren eingesetzte Druck ist ebenfalls unkritisch, wobei dieser vielfach von der Art des bioaktiven Naturstoffs und der Verfestigungstemperatur des Verkapselungsmaterials abhängig ist. Beispielsweise kann der Druck im Bereich von 10 mbar bis 200 bar, bevorzugt im Bereich von 100 mbar bis 100 bar gewählt werden.

Nach dem Abtrennen können die erhaltenen Partikel gewaschen werden. Hierzu können die Partikel mit einer Waschflüssigkeit behandelt werden, um Additivreste und/oder bioaktive Naturstoffe, die sich auf der Oberfläche der Partikel befindet von den Partikeln zu trennen. Dementsprechend sollten die Partikel, insbesondere das Verkapselungsmaterial nicht in der Waschflüssigkeit löslich sein. Andererseits sollte die abzutrennende Substanz, beispielsweise der bioaktive Naturstoff eine möglichst hohe Löslichkeit aufweisen. Zu den bevorzugten Waschflüssigkeiten zählen insbesondere Wasser und/oder Alkohole mit 1 bis 7, vorzugsweise 1 bis 4 Kohlenstoffatomen, insbesondere Methanol, Ethanol, Propanol und/oder Butanol. Diese Flüssigkeiten können einzeln oder auch als Mischung von zwei, drei oder mehr Flüssigkeiten eingesetzt werden.

Die Temperatur beim Waschen kann ebenfalls in einem weiten Bereich liegen, wobei diese unter anderem von der Verfestigungstemperatur des Verkapselungsmaterials abhängig ist. Um eine Aggregation der Partikel zu vermeiden, sollte die gewählte Temperatur unterhalb der Verfestigungstemperatur des Verkapselungsmaterials liegen. Vorzugsweise liegt die Temperatur im Bereich von -20°C bis 100°C, besonders bevorzugt im Bereich von -10°C bis 40°C. Der beim Waschen eingesetzte Druck ist ebenfalls unkritisch, wobei dieser vielfach von der Art des bioaktiven Naturstoffs und der Verfestigungstemperatur des Verkapselungsmaterials abhängig ist. Beispielsweise kann der Druck beim Waschen im Bereich von 10 mbar bis 200 bar, bevorzugt im Bereich von 100 mbar bis 100 bar gewählt werden.

Die zum Waschen der Partikel verwendbaren Vorrichtungen sind allgemein bekannt. So können hierfür beispielsweise Vorrichtungen eingesetzt werden, die ein Mischgefäß und einen Separator umfassen. Die Mischgefäße umfassen vorzugsweise die zuvor dargelegten Geräten und Vorrichtungen zum Dispergieren.

In einem weiteren Schritt können die erhaltenen Mikropartikel getrocknet werden. Die zum Trocknen der Mikropartikel verwendbaren Vorrichtungen sind allgemein bekannt. So können unter anderem Trommeltrockner, Taumeltrockner, Teller-trockner, Schneckentrockner, Schaufeltrockner, Zylindertrockner, Walzentrockner, Gefriertrockner, Wirbelschichttrockner, Sprühtrockner, Stromtrockner, Mahltrockner, Hordentrockner, Tunneltrockner, Vakuumtrockner- und/oder Vakuumkontakttrockner eingesetzt werden.

Die Temperatur beim Trocknen kann ebenfalls in einem weiten Bereich liegen, wobei diese unter anderem von der Verfestigungstemperatur des Verkapselungsmaterials abhängig ist. Um eine Aggregation der Partikel zu vermeiden, sollte die gewählte Temperatur unterhalb der Verfestigungstemperatur des Verkapselungsmaterials liegen. Vorzugsweise liegt die Temperatur beim Trocknen im Bereich von -20°C bis 50°C, besonders bevorzugt im Bereich von - 10°C bis 30°C. Der beim Trocknen eingesetzte Druck ist ebenfalls unkritisch, wobei dieser vielfach von der Art des bioaktiven Wirkstoffs und der Verfestigungstemperatur des Verkapselungsmaterials abhängig ist. Beispielsweise kann der Druck im Bereich von 0,1 mbar bis 10 bar, bevorzugt im Bereich von 0,2 mbar bis 2 bar gewählt werden.

Das zuvor beschriebene Verfahren können mit einfachen Anlagen durchgeführt werden, die aus an sich bekannten Komponenten aufgebaut sein können. Geeignete Anlagen umfassen vorzugsweise mindestens zwei Mischgefäße und einen Separator, wobei die Mischgefäße über mindestens eine Zuführung miteinander verbunden sind und das zweite Mischgefäß mit dem Separator verbunden ist. Die im Separator abgetrennte zweite Phase kann vorzugsweise über eine Rückführung in ein Mischgefäß zurückgeführt werden.

Gemäß einer bevorzugten Ausführungsform kann in der Leitung zwischen dem ersten Mischgefäß, in dem die Schmelze hergestellt wird und dem zweiten Mischgefäß, in dem die Schmelze in der zweiten flüssigen Phase dispergiert wird, eine Pumpe vorgesehen sein, die für hochviskose Flüssigkeiten geeignet ist. Zu den bevorzugten Pumpen gehören insbesondere Schraubenpumpen, beispielsweise Schraubenpumpen mit einer, zwei oder drei Schrauben; Schraubenverdichter, Flügelpumpen, Drehkolbenpumpen, Rotationspumpen, Kolbenpumpen, Rotationskolbenpumpen und/oder Schlauchpumpen.

Gemäß einem besonderen Aspekt der vorliegenden Erfindung weist die Anlage vorzugsweise mindestens drei Mischgefäße auf, wobei mindestens zwei Mischgefäße über Zuführungen mit mindestens einem Mischgefäß verbunden sind. Hierbei dient mindestens ein Mischgefäß zum Herstellen der Schmelze, mindestens ein Mischgefäß zum Herstellen der zweiten flüssigen Phase und mindestens ein Mischgefäß zum Dispergieren der Schmelze in der zweiten flüssigen Phase. Die Schmelze und die zweite flüssige Phase können in weiteren separaten Mischgefäßen chargenweise oder kontinuierlich hergestellt werden, um eine kontinuierliche Produktion von Mikropartikeln sicherzustellen.

Die in der Anlage zur Herstellung von erfindungsgemäßen Präparaten eingesetzten Mischgefäße können temperierbar ausgestattet sein. Dementsprechend können diese Mischgefäße Heizelemente oder Kühlelemente umfassen.

Vorzugsweise kann die Anlage mindestens einen Trockner aufweisen, der mit dem Separator verbunden ist. Des Weiteren kann die Anlage bevorzugt eine Vorrichtung zum Waschen von Partikeln umfassen.

Das Verfestigen der Schmelze in der Dispersion kann in der Anlage über verschiedene Maßnahmen erzielt werden. Beispielsweise kann das Mischgefäß, in dem die Dispersion hergestellt wurde, abgekühlt werden. Dies kann beispielsweise durch eine externe Kühlung oder durch Zuleiten von Flüssigkeiten erfolgen, die vorzugsweise die gleiche oder eine ähnliche Zusammensetzung aufweist wie die zweite flüssige Phase. Bevorzugt kann hierfür auch ein Wärmeüberträger bzw. Wärmetauscher, ein Mischventil oder ein zusätzliches Mischgefäß eingesetzt werden.

Die Anlage kann Pumpen umfassen, die beispielsweise für den Transport von Flüssigkeiten oder zum Erzeugen von Über- bzw. Unterdruck dienen können. Geeignete Pumpen sind vom jeweiligen Zweck abhängig. Zu den bevorzugten Pumpen gehören beispielsweise Verdrängerpumpen, wie zum Beispiel Schöpfwerke, Förderschnecken, Balgpumpen, Kolbenpumpen, Rotationskolbenpumpen, Zahnradpumpen außen/innenverzahnt, Membranpumpen, Drehschieberpumpen, Zentrifugalpumpe, Schlauchpumpen, Zahnriemenpumpen, Exzenterschneckenpumpen, Schraubenpumpen und Schraubenverdichter und/oder Hydraulischer Widder; Strömungspumpen, wie zum Beispiel Kreiselpumpe, Axialpumpe, Diagonalpumpe und/oder Radialpumpe; Blasenpumpe, Wasserstrahlpumpe, Dampfstrahlpumpe, Stoßheber (Hydraulischer Widder), Pferdekopfpumpe (Tiefpumpe); Vakuumpumpen, wie zum Beispiel Verdrängerpumpe, Treibmittelpumpe, Molekularpumpe, Turbomolekularpumpe, Kryopumpe, Sorptionspumpe, Öldiffusionspumpe.

Derartige Anlagen werden in den nachfolgend näher beschriebenen Figuren beispielhaft erläutert.

Figur 1 beschreibt eine erste Ausführungsform einer Anlage zur Durchführung des Verfahrens der vorliegenden Erfindung.

Figur 2 beschreibt eine zweite Ausführungsform einer Anlage zur Durchführung des Verfahrens der vorliegenden Erfindung.

Figur 3 beschreibt eine dritte Ausführungsform einer Anlage zur Durchführung des Verfahrens der vorliegenden Erfindung.

Figur 4 beschreibt eine vierte Ausführungsform einer Anlage zur Durchführung des Verfahrens der vorliegenden Erfindung.

Figur 5 beschreibt eine fünfte Ausführungsform einer Anlage zur Durchführung des Verfahrens der vorliegenden Erfindung.

Figur 6 beschreibt eine sechste Ausführungsform einer Anlage zur Durchführung des Verfahrens der vorliegenden

Erfindung.

In Figur 1 ist eine erste Ausführungsform einer Anlage zur Durchführung des Verfahrens der vorliegenden Erfindung dargestellt. Diese Anlage kann beispielsweise ein, zwei oder mehr Zuführungen 1 bzw. 2, beispielsweise Leitungen oder Zuführschnecken, aufweisen, über die ein oder mehrere Polymere mit Polyestereinheiten, ein oder mehrere Glyceride und/oder ein oder mehrere bioaktive Naturstoffe einem ersten Mischgefäß 3 zugeführt werden. Im Mischgefäß 3 können die zugeführten Substanzen in eine Schmelze überführt werden, die mindestens ein Polymere mit Polyestereinheiten, mindestens ein Glycerid und mindestens einen bioaktiven Naturstoff umfasst. In Mischgemäß 3 können die Komponenten fein ineinander verteilt werden. So kann beispielsweise eine Lösung, eine Dispersion oder Suspension hergestellt werden. Vielfach bildet das Verkapselungsmaterial die Matrixphase, in der der bioaktive Naturstoff verteilt wird. Hierfür können die zuvor beschriebenen Vorrichtungen eingesetzt werden.

Die in Mischgefäß 3 erhaltene Schmelze kann beispielsweise mit einer Pumpe 4 über die Zuführung 5, beispielsweise eine Leitung, in das Mischgefäß 6 überführt werden.

Das Mischgefäß 6 kann ein, zwei, drei, vier oder mehr weitere Zuführungen 7, 8, 9, 10, beispielsweise Leitungen oder Zuführschnecken, aufweisen, über die beispielsweise Stabilisatoren, Emulgatoren, Warmwasser und/oder Kaltwasser zugeführt werden können. In der vorliegenden Figurenbeschreibung wird Wasser beispielhaft als zweite flüssige Phase eingesetzt. Allerdings ist dem Fachmann offensichtlich, dass jede andere zuvor als Hauptbestandteil der zweiten flüssigen Phase beschriebene Verbindung ebenfalls anstatt oder zusammen mit Wasser eingesetzt werden kann. Das Wasser dient somit lediglich als Beispiel für die zuvor dargelegten Verbindungen, das entsprechend durch die anderen Substanzen ersetzt werden kann.

Die Zuführungen 7, 8, 9, 10 können alle im Mischgefäß 6 münden. Des Weiteren können diese Zuführungen auch vor dem Eintritt in das Mischgefäß 6 zusammengeführt werden.

Vor der Zuführung der Schmelze in das Mischgefäß 6 kann beispielsweise über die Zuführungen 7, 8 und 9 eine Lösung hergestellt werden, die als Hauptbestandteil beispielsweise Wasser oder Ethanol sowie Hilfsstoffe, beispielsweise Stabilisatoren und Emulgatoren umfasst. Diese Lösung kann auf eine Temperatur oberhalb der Verfestigungstemperatur des Verkapselungsmaterials erwärmt werden. Des Weiteren können die zugeführten Komponenten bereits eine entsprechende Temperatur aufweisen.

Nach Herstellung einer entsprechenden Lösung in Mischgefäß 6 kann die in Mischgefäß 3 hergestellte Schmelze dem Mischgefäß 6 zugeführt werden. In Mischgefäß 6 wird die Schmelze in der zuvor beschriebenen Lösung dispergiert. Hierfür weist das Mischgefäß 6 bekannte Vorrichten zum Dispergieren auf. Hierfür können die zuvor beschriebenen Vorrichtungen eingesetzt werden.

Nach dem durch das Dispergieren die gewünschte Tropfengröße und Tropfengrößenverteilung erhalten wurde, kann die in der zweiten flüssigen Phase, beispielsweise Wasser, dispergiert vorliegende Schmelze verfestigt werden. Hierzu kann beispielsweise über eine Zuführung 10 Kaltwasser in das Mischgefäß 6 eingeleitet werden. Des Weiteren kann das Mischgefäß 6 mittels eines Kühlmediums, das durch einen Wärmeüberträger oder einen Doppelmantel geleitet wird, abgekühlt werden.

Die so erhaltenen Partikel können aus der zweiten flüssigen Phase abgetrennt werden. Hierzu kann die in Mischgefäß 6 erhaltene Zusammensetzung, die verfestigte Mikropartikel aufweist, beispielsweise mit einer Pumpe 11 über die Leitung 12 in den Separator 13 überführt werden. Der Separator 13 dient zum Abtrennen oder Aufkonzentrieren der in der zweiten flüssigen Phase enthaltenen Mikropartikel, wobei jede der zuvor dargelegten Vorrichtungen hierfür eingesetzt werden kann. Vorliegend werden im Separator die Mikropartikel von der zweiten flüssigen Phase getrennt, wobei vielfach eine Aufkonzentration ausreichend sein kann. Die abgetrennte zweite flüssige Phase, die beispielsweise Wasser, Emulgatoren und Stabilisatoren umfassen kann, kann über eine Rückführung 14, beispielsweise eine Leitung in das Mischgefäß 6 eingeleitet werden.

Die abgetrennten Mikropartikel können beispielsweise mit einer Pumpe 15 über die Zuführung 16, beispielsweise eine Leitung, in den Trockner 17 überführt werden. Im Trockner 17 können Reste der zweiten flüssigen Phase, beispielsweise Wasser entfernt werden. Die getrockneten Mikropartikel können über die Leitung 18 dem Trockner entnommen werden. Im Folgenden wird unter Bezugnahme auf die Figur 2 eine zweite Ausführungsform einer Anlage zur Durchführung des Verfahrens der vorliegenden Erfindung beschrieben, die der ersten Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Wie auch die erste Ausführungsform weist auch die zweite Ausführungsform ein Mischgefäß 3 mit Zuführungen 1, 2 zur Herstellung einer Schmelze, ein Mischgefäß 6 zum Dispergieren der Schmelze in einer zweiten flüssigen Phase, einen Separator 13 und einen Trockner 17 auf.

In der zweiten Ausführungsform wird die zweite flüssige Phase jedoch in einem weiteren Mischgefäß 19 hergestellt, das beispielsweise ein, zwei, drei oder mehr Zuführungen 20, 21, 22 aufweisen kann. Über die Zuführungen 20, 21 und 22 können die Komponenten der zweiten flüssigen Phase, als Hauptbestandteil beispielsweise Wasser oder Ethanol sowie Hilfsstoffe, beispielsweise Stabilisatoren und Emulgatoren in das Mischgefäß 19 zugegeben werden.

Auch in diesem Beispiel kann das Wasser oder das Ethanol unabhängig von den weiteren Komponenten durch jeden der zuvor dargelegten Verbindungen der zweiten flüssigen Phase ersetzt werden.

Die in Mischgefäß 19 erhaltene Lösung kann beispielsweise mit einer Pumpe 23 über die Zuführung 9, beispielsweise eine Leitung, in das Mischgefäß 6 überführt werden. In das Mischgefäß 6 kann beispielsweise über die Zuführung 10 Kaltwasser bzw. abgekühltes Ethanol eingeleitet werden, um die dispergierte Schmelze zu verfestigen. Über die Rückführung 14 kann die im Separator 13 abgetrennte zweite flüssige Phase, beispielsweise Wasser oder Ethanol, die zusätzlich Hilfsstoffe, wie Emulgatoren oder Stabilisatoren enthalten kann, in das Mischgefäß 6 zurückgeführt werden. Somit können von Mischgefäß 19 lediglich die Mengen an zweiter flüssiger Phase in das Mischgefäß 6 eingeleitet werden, die nicht im Separator 13 zurück gewonnen werden können.

Die weiteren Komponenten der Anlage entsprechen im Wesentlichen denen der ersten Ausführungsform, so dass hieraus Bezug genommen wird.

Im Folgenden wird unter Bezugnahme auf die Figur 3 eine dritte Ausführungsform einer Anlage zur Durchführung des Verfahrens der vorliegenden Erfindung beschrieben, die der zweiten Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Wie auch die zweite Ausführungsform weist auch die dritte Ausführungsform ein Mischgefäß 3 mit Zuführungen 1, 2 zur Herstellung einer Schmelze, ein Mischgefäß 19 zur Herstellung der zweiten flüssigen Phase, ein Mischgefäß 6 zum Dispergieren der Schmelze in einer zweiten flüssigen Phase, einen Separator 13 und einen Trockner 17 auf.

In der dritten Ausführungsform wird die Abkühlung der Schmelze nach dem Dispergieren in der zweiten flüssigen Phase durch eine externe Kühlung 24, vorzugsweise einen Wärmetauscher, erzielt, die in Leitung 12 zwischen dem Mischgefäß 6 und dem Separator 13 vorgesehen ist. In dem Wärmetauscher 24 erfolgt die Verfestigung der dispergierten Schmelze.

Durch diese besondere Ausgestaltung kann das vorliegende Verfahren auch kontinuierlich durchgeführt werden. Des Weiteren kann diese Ausführungsform besonders energiesparend betrieben werden.

Die weiteren Komponenten der Anlage entsprechen im Wesentlichen denen der zweiten Ausführungsform, so dass hieraus Bezug genommen wird.

Im Folgenden wird unter Bezugnahme auf die Figur 4 eine vierte Ausführungsform einer Anlage zur Durchführung des Verfahrens der vorliegenden Erfindung beschrieben, die der zweiten Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Wie auch die zweite Ausführungsform weist auch die vierte Ausführungsform ein Mischgefäß 3 mit Zuführungen 1, 2 zur Herstellung einer Schmelze, ein Mischgefäß 19 zur Herstellung der zweiten flüssigen Phase, ein Mischgefäß 6 zum Dispergieren der Schmelze in einer zweiten flüssigen Phase, einen Separator 13 und einen Trockner 17 auf.

In der vierten Ausführungsform wird die Abkühlung der Schmelze nach dem Dispergieren in der zweiten flüssigen Phase durch das Zuleiten einer kalten Flüssigkeit über Leitung 25 erzielt, die im Wesentlichen der Zusammensetzung der zweiten flüssigen Phase entspricht, um die Schmelze zu verfestigen. Die Zuleitung der kalten Flüssigkeit kann über ein Mischventil 26 erfolgen, das in Leitung 12 zwischen dem Mischgefäß 6 und dem Separator 13 vorgesehen ist.

Über die Rückführung 14 kann ein Teil der im Separator 13 abgetrennten zweiten flüssigen Phase, beispielsweise Wasser oder Ethanol, die zusätzlich Hilfsstoffe, wie Emulgatoren oder Stabilisatoren enthalten kann, in das Mischgefäß 6 zurückgeführt werden. Somit können von Mischgefäß 19 lediglich die Mengen an zweiter flüssiger Phase in das Mischgefäß 6 eingeleitet werden, die nicht im Separator 13 zurück gewonnen werden können. Hierbei kann dieser Teil auf die Temperatur des Mischgefäßes 6 erwärmt werden. Ein weiterer Teil der im Separator 13 abgetrennten zweiten flüssigen Phase kann in die Leitung 25 geleitet werden. Hierbei kann die zweite Phase abgekühlt werden, so dass die Temperatur der in die Leitung 25 eingeleitet zweite flüssige Phase der Temperatur der kalten Flüssigkeit entspricht.

Durch diese besondere Ausgestaltung kann das vorliegende Verfahren auch kontinuierlich durchgeführt werden. Des Weiteren kann diese Ausführungsform besonders energiesparend betrieben werden.

Die weiteren Komponenten der Anlage entsprechen im Wesentlichen denen der zweiten Ausführungsform, so dass hieraus Bezug genommen wird.

Im Folgenden wird unter Bezugnahme auf die Figur 5 eine fünfte Ausführungsform einer Anlage zur Durchführung des Verfahrens der vorliegenden Erfindung beschrieben, die der zweiten Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Wie auch die zweiten Ausführungsform weist auch die fünfte Ausführungsform ein Mischgefäß 3 mit Zuführungen 1, 2 zur Herstellung einer Schmelze, ein Mischgefäß 19 zur Herstellung der zweiten flüssigen Phase, ein Mischgefäß 6 zum Dispergieren der Schmelze in einer zweiten flüssigen Phase, einen Separator 13 und einen Trockner 17 auf.

In der fünften Ausführungsform wird die Abkühlung der Schmelze nach dem Dispergieren in der zweiten flüssigen Phase in einem Mischgefäß 27 durchgeführt, wobei die Abkühlung beispielsweise durch Zuleiten einer kalten

Flüssigkeit erzielt werden kann, die im Wesentlichen der Zusammensetzung der zweiten flüssigen Phase entspricht, um die Schmelze zu verfestigen. Die Zuleitung der kalten Flüssigkeit kann über die Leitung 28 erfolgen.

Über die Rückführung 14 kann ein Teil der im Separator 13 abgetrennten zweiten flüssigen Phase, beispielsweise Wasser oder Ethanol, die zusätzlich Hilfsstoffe, wie Emulgatoren oder Stabilisatoren enthalten kann, in das Mischgefäß 6 zurückgeführt werden. Somit können von Mischgefäß 19 lediglich die Mengen an zweiter flüssiger Phase in das Mischgefäß 6 eingeleitet werden, die nicht im Separator 13 zurück gewonnen werden können. Hierbei kann dieser Teil auf die Temperatur des Mischgefäßes 6 erwärmt werden. Ein weiterer Teil der im Separator 13 abgetrennten zweiten flüssigen Phase kann in die Leitung 28 geleitet werden. Hierbei kann die zweite Phase abgekühlt werden, so dass die Temperatur der in die Zuleitung 28 eingeleitet zweite flüssige Phase der Temperatur der kalten Flüssigkeit entspricht.

Durch diese besondere Ausgestaltung kann das vorliegende Verfahren auch kontinuierlich durchgeführt werden. Des Weiteren kann diese Ausführungsform besonders energiesparend betrieben werden.

Die weiteren Komponenten der Anlage entsprechen im Wesentlichen denen der zweiten Ausführungsform, so dass hieraus Bezug genommen wird.

Im Folgenden wird unter Bezugnahme auf die Figur 6 eine sechste Ausführungsform einer Anlage zur Durchführung des Verfahrens der vorliegenden Erfindung beschrieben, die der fünften Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Wie auch die fünfte Ausführungsform weist auch die sechste Ausführungsform ein Mischgefäß 3 mit Zuführungen 1, 2 zur Herstellung einer Schmelze, ein Mischgefäß 19 zur Herstellung der zweiten flüssigen Phase, ein Mischgefäß 6 zum Dispergieren der Schmelze in einer zweiten flüssigen Phase, einen Separator 13 und einen Trockner 17 auf. Hierbei kann die Überführung der in dem Mischgefäß 6 erhaltenen Zusammensetzung in das Mischgefäß 27 durch eine Pumpe 38 unterstützt werden, die in Leitung 12 vorgesehen ist.

Die sechste Ausführungsform weist ein Mischgefäß 29 auf, in der die Schmelze vorgemischt werden kann, um in Mischgefäß 3 einen Füllstand zu gewährleisten, der einen kontinuierlichen Zufluss an Schmelze in das Mischgefäß 6 sicherstellt. Im Mischgefäß 29 wird kontinuierlich oder in Chargen die Schmelze gebildet, wobei in das Mischgefäß 29 das Verkapselungsmaterial und der bioaktive Naturstoff über die Zuführungen 1 und 2 zugeführt werden können. Die Schmelze kann mit einer Pumpe 30 über Leitung 31 in das Mischgefäß 3 überführt werden.

Des Weiteren kann auch die zweite flüssige Phase in einem Mischgefäß 32 vorgebildet werden, bevor die zweite flüssige Phase in das Mischgefäß 19 überführt wird. Durch diese Maßnahme kann sichergestellt werden, dass aus Mischgefäß 19 kontinuierlich zweite flüssige Phase in das Mischgefäß 6 überführt wird. In Mischgefäß 32 kann die zweite flüssige Phase kontinuierlich oder in Chargen gebildet werden, wobei die Komponenten über die Zuleitungen 33, 34 und 35 in das Mischgefäß 32 eingeleitet werden können. Die hergestellte zweite flüssige Phase kann mit einer Pumpe 36 über Leitung 37 in das Mischgefäß 19 überführt werden.

Über die Rückführung 14 kann ein Teil der im Separator 13 abgetrennten zweiten flüssigen Phase, beispielsweise Wasser oder Ethanol, die zusätzlich Hilfsstoffe, wie Emulgatoren oder Stabilisatoren enthalten kann, in das Mischgefäß 32 oder 19 zurückgeführt werden. Des Weiteren kann ein Teil der im Separator 13 abgetrennten zweiten flüssigen Phase über die Rückführung in das Mischgefäß 6 geleitet werden (nicht gezeigt). Somit können von Mischgefäß 19 lediglich die Mengen an zweiter flüssiger Phase in das Mischgefäß 6 eingeleitet werden, die nicht im Separator 13 zurück gewonnen werden können. Hierbei kann dieser Teil auf die Temperatur des Mischgefäßes 6 bzw. 32 oder 19 erwärmt werden.

Ein weiterer Teil der im Separator 13 abgetrennten zweiten flüssigen Phase kann in die Zuleitung 28 geleitet werden. Hierbei kann die zweite Phase abgekühlt werden, so dass die Temperatur der in die Zuleitung 28 eingeleitet zweite flüssige Phase der Temperatur der kalten Flüssigkeit entspricht.

Die sechste Ausführungsform weist eine Vorrichtung zum Waschen der Partikel auf. Diese Vorrichtung umfasst ein Mischgefäß sowie einen Separator, wobei diese Bauteile auch in einem Gehäuse untergebracht sein können. Dementsprechend können die in Separator 13 abgetrennten Partikel mit einer Pumpe 39 über Zuführung 40 in ein Mischgefäß 41 überführt werden, in dem die Partikel mit einer Waschflüssigkeit gereinigt werden können, die über Leitung 42 zugeführt wird. Mit einer Pumpe 43 kann die Zusammensetzung über Leitung 44 in einen Separator 45 überführt werden. Im Separator 45 werden die gereinigten Partikel von der Waschflüssigkeit getrennt. Die Waschflüssigkeit kann aufbereitet und wieder verwendet werden, wobei die Rückführung 46, je nach Art der Waschflüssigkeit, sowohl in die Leitung 42 zurückgeführt werden kann (nicht gezeigt) oder in eines der zuvor verwendeten Mischgefäße 6, 19, 27 und/oder 32, wobei die Temperatur der Waschflüssigkeit jeweils angepasst werden kann. Die gereinigten Partikel können mit Pumpe 47 über Zuführung 48 in den Trockner 17 überführt werden.

Durch diese besondere Ausgestaltung kann das vorliegende Verfahren auch kontinuierlich durchgeführt werden. Des Weiteren kann diese Ausführungsform besonders energiesparend betrieben werden. Darüber hinaus können Partikel mit einem besonders günstigen und steuerbaren Freisetzungsprofil erhalten werden.

Die weiteren Komponenten der Anlage entsprechen im Wesentlichen denen der fünften Ausführungsform, so dass hieraus Bezug genommen wird.

Die erfindungsgemäßen Präparate können beispielsweise in Kosmetika, in Arzneimitteln, in Deodorants, in Nahrungsmitteln, in Futtermitteln, in Getränken, in feuchtigkeitsspendenden Zusammensetzungen, wie beispielsweise Emollients und/oder Moisturisers, in Phytonutrients und/oder in Verpackungen eingesetzt werden. Der Begriff Emollients ist an sich weit verbreitet und bezeichnet im Allgemeinen ein kosmetisches Öl, dem eine feuchtigkeitsspende Eigenschaft zugesprochen wird. Solche Öle werden teilweise zur Behandlung von trockener Haut eingesetzt. Unter Phytonutrients werden insbesondere Nahrungsmittelzusätze auf pflanzlicher Basis verstanden, die vorteilhafte Wirkungen aufweisen. Diese Nahrungsmittelzusätze können beispielsweise die zuvor dargelegten Carotinoide, Flavonoide, Phytosterole und/ oder Polyphenole enthalten.

Nachfolgend soll die vorliegende Erfindung anhand von Beispielen erläutert werden, ohne dass hierdurch eine Beschränkung erfolgen soll.

Beispiele

Nachweis des enzymatischen Abbaus von Boltorn® H30 und Boltorn® H40

Der Abbau der Moleküle Boltorn®H30 und Boltorn® H40 (Perstorp) in wässrigen, enzymhaltigen Lösungen wurde durch nachstehende Experimente gezeigt:

Die Polymere Boltorn® H30 und Boltorn® H40 (Perstop) wurden in separaten Versuchen in einer elektrischen Mühle gemahlen und gesiebt. Weiterhin wurde mit der Partikelfraktion zwischen 90 und 250 μm gearbeitet. Die Polymerpartikel wurden in einer Lösung von Lipase aus Candida cylindracea, 0,5 mg/ml (Lipase Lipomod® 34P, Biocatalyst Ltd., UK) und Phosphatpuffer, pH = 5, bei 37°C suspendiert. Als Kontrollprobe wurde reiner Puffer bei den gleichen Bedingungen verwendet. Die Konzentration des Monomers 2,2-bis-Hydroxymethylpropionsäure der hyperverzweigten Polymere Boltorn® H30 und Boltorn® H40 wurde mit Hilfe der UV-Spektroskopie analysiert (Peak bei 208.5 nm). Die nachgewiesenen Mengen an Monomer sind in Tabellen 1 und 2 zusammengefasst.

Die Konzentration der Hydroxymethylpropionsäure in der lipasehaltigen Lösung ist nach 24 Stunden um den Faktor 4,7 bzw. 4,8 höher als die Konzentration in reinem Puffer. Die Polymere Boltorn® H30 und Boltorn® H40 sind somit enzymatisch abbaubare hyperverzweigte Polymere.

Tabelle 1

| Abbau von Boltorn® H30 | | |
|---|---|---|
| Zeit in h | Konzentration von Hydroxymethylpropionsäure (g/ml) | |
| | Puffer ohne Enzym | Puffer mit Enzym |
| 0 | 0,045 | 0, 045 |
| 24 | 0,088 | 0,248 |

Tabelle 2

| Abbau von Boltorn® H40 | | |
|---|---|---|
| Zeit in h | Konzentration von Hydroxymethylpropionsäure (g/ml) | |
| | Puffer ohne Enzym | Puffer mit Enzym |
| 0 | 0,047 | 0,047 |
| 24 | 0,093 | 0,270 |

**Vergleichsbeispiel 1 (nicht erfindungsgemäß)**

Unter Verwendung der in Abbildung 1 dargestellten Anlage wurde ein Präparat hergestellt, welches Kernöl aus Pfirsichsteinen und einen hyperverzweigten Polyester umfasste.

Der eingesetzte hyperverzweigte Polyester wurde durch Hydrophobisierung eines hydrophilen hyperverzweigten Polyesters, der ein Gewichtsmittel des Molekulargewichts Mw von 3500 g/mol, eine Glastemperatur von etwa 35°C und eine Hydroxy-Zahl von etwa 490 mg KOH/g aufwies, erhalten (kommerziell erhältlich von der Firma Perstorp unter

der Bezeichnung Boltorn® H30). Die Hydrophobisierung erfolgte durch Veresterung des hydrophilen Polymers mit einer Mischung aus Stearinsäure und Palmitinsäure (massenbezogenes Verhältnis von Stearinsäure zu Palmitinsäure = 2 zu 1), wobei 90% der Hydroxygruppen des hydrophilen Polymers umgesetzt wurden. Das Molekulargewicht MW betrug 10000 g/mol. Die Veresterung wurde wie in WO 93/17060 beschrieben durchgeführt. Der hydrophobisierte hyperverzweigte Polyester hatte einen Schmelzpunkt von 49°C.

Zur Herstellung des Präparates wurden 10 Gew.-% Pfirsichkernöl in dem geschmolzenen Polymer bei einer Temperatur von etwa 60°C mit einem Spiral-Rührer bei 200 Umdrehungen pro Minute in einem ersten Mischgefäß (Gefäß mit dem Bezugszeichen 3 in Figur 1) 5 Minuten gelöst.

Im einem weiteren Mischgefäß wurde eine Mischung aus Tensiden bestehend aus 1 Gew.-% Polyvinylalkohol (M = 6000 g/mol, Polisciences®, Warrington, USA) und 0,1 Gew.-% eines ethoxylierten Fettalkohols (Tego® Alkanol L4 der Firma Degussa GmbH) in Wasser bei 60 °C unter Rühren vorgelegt. Diese Mischung fungiert als kontinuierliche Phase.

Anschließend wurde ein Gewichtsteil der im ersten Mischgefäß hergestellten Schmelze, die neben dem Polymer auch das Kernöl enthielt, aus dem ersten Mischgefäß unter fortwährendem Rühren mit einem ULTRA-TURRAX® Rührer bei 8.000 Umdrehungen pro Minute zu 9 Gewichtsteilen der kontinuierlichen Phase in ein zweites Mischgefäß (Gefäß mit dem Bezugszeichen 6 in Figur 1) bei 60°C gegeben. Nach einer Verweilzeit von 0,2 bis 10 Minuten und einer Erniedrigung der Systemtemperatur im zweiten Mischgefäß auf eine Temperatur, die 15 °C unterhalb der Schmelztemperatur des Polymers liegt, bilden sich Partikel. Mit einer Schlauchpumpe wurde die Suspension einer Zentrifuge zugeleitet, in der bei 25°C die Wirkstoffpartikel von der kontinuierlichen Phase abgetrennt und mit Wasser gewaschen wurden. Anschließend wurden die Wirkstoffpartikel in einem Vakuumtrockner bei 20°C und 10 mbar für 100 h getrocknet.

Die erhaltenen Partikel zeigen eine Partikelgrößenverteilung von 5 $\mu$m < $d_{P90}$ < 50 $\mu$m und bestehen aus dem hyperverzweigten fettsäuremodifizierten Polyester und ca. 9,9 Gew.-% Kernöl (bezogen auf die Partikelmasse).

Nachfolgend wurden die Eigenschaften der Partikel untersucht. Hierzu wurden insbesondere die Lagerstabilität, die Stabilität der Partikel in einer Pufferlösung sowie die Freisetzung des Wirkstoffes durch enzymatischen Abbau bestimmt.

Die Lagerstabilität wurde durch iodometrische Titration bestimmt. Hierzu wurde 0,1 g Kernöl in 10 ml Phosphatpufferlösung (pH = 5) in zwei verschiedene 300 ml Kolben (Probe KO1 und KO2) dispergiert. Das Kernöl im Kolben KO1 wurde mit 10ml Chloroform gelöst. Zu der Lösung wurden genau 10 ml der Iodmonobromid-Reagenzlösung pipettiert. Der Kolben wurde verschlossen, kurz umgeschüttelt und 1 Stunde im Dunkeln stehen gelassen. Anschließend wurden 20 ml Kaliumiodid-Lösung und 100 ml Wasser zugefügt. Das freigesetzt Iod wurde mit Natriumthiosulfatlösung gegen Stärke zurücktitriert. Nach 24 Stunden wurde die Probe KO2 ebenfalls nach dieser Vorgehensweise titriert. Die Iodzahl wurde mit der nachstehenden Gleichung berechnet:

$$IZ = (a-b) \cdot 12{,}69 \cdot 100/(m \cdot 1000)$$

wobei

IZ = Iodzahl [g Iod/100g Öl]
a = Verbrauch an Natriumthiosulfatlösung im Blindversuch [ml]
b = Verbrauch an Natriumthiosulfatlösung bei der jeweiligen Probe [ml]
m = Einwaage an Kernöl [g]

In gleicher Weise wurde die Iodzahl für 1g der Partikel aus Vergleichsbeispiel 1 bestimmt.

Die Ergebnisse sind in Tabelle 3 zusammengefasst.

Tabelle 3

| Lagerstabilität des Präparats aus Kernöl und hyperverzweigtem Polyester | | |
|---|---|---|
| Zeit in h | Iodzahl [g Iod/100g Einwaage] | |
| | Partikel aus Vergleichsbeispiel 1 | Reines Kernöl |
| 0 | 9,3 | 98,6 |
| 24 | 9,2 | 97,0 |

Die Freisetzung von Kernöl aus den Partikeln von Vergleichsbeispiel 1 wurde in einem Puffer ohne Enzym bei

pH 5,0 und 37°C durch die zuvor dargelegte iodo-metrische Titration bestimmt. Sie betrug nach 2 Stunden 5,6% und nach 4 Stunden 36,0%, jeweils bezogen auf den Gesamtgehalt der Partikel an Naturstoff. Durch Verwendung einer Pufferlösung mit Lipase (Lipomod® 34P; die Konzentration des Lipomods 34P betrug 0.5 mg/ml) erhöhen sich die zuvor genannten Werte auf 9,9% nach 2 Stunden bzw. 52,7% nach 4 Stunden.

**Beispiel 1**

Das Vergleichsbeispiel 1 wurde im Wesentlichen wiederholt. Allerdings wurden zur Herstellung des Präparates 10 Gew.-% Pfirsichkernöl bei einer Temperatur von etwa 60°C mit einem Spiral-Rührer bei 200 Umdrehungen pro Minute in einem ersten Mischgefäß 5 Minuten in einer Zusammensetzung gelöst, die 50 Gew.-% Polymer und 50 Gew.-% Triglycerid umfasst. Das Triglycerid auf pflanzlicher Basis weist einen Schmelzpunkt von 57 - 60°C, eine Dichte von 0,877 g/cm$^3$ bei 60°C und eine dynamische Viskosität von 18 mPa.s bei 70°C auf (kommerziell erhältlich unter der Handelsbezeichnung Tegin® BL 150 V Roh von der Firma Goldschmidt GmbH).

Die erhaltenen Partikel zeigen eine Partikelgrößenverteilung von 5 $\mu$m < $d_{P90}$< 50 $\mu$m und bestehen aus dem Verkapselungsmaterial, das neben dem hyperverzweigten, fettsäuremodifizierten Polyester zusätzlich Triglycerid umfasst, und ca. 9,8 Gew.-% Kernöl (bezogen auf die Partikelmasse).

Das zuvor erhalten Präparat zeigte eine hervorragende Lagerstabilität. Eine iodometrische Titration zeigte nach einer Lagerung von 24 h keinen Abbau des Kernöls. So wies das Präparat unmittelbar nach der Herstellung und nach 24 Stunden Lagerung bei 25°C jeweils eine Iodzahl von 9,3 [g Iod/ 100g Formulierung] auf.

Die Freisetzung von Kernöl aus den Partikeln von Beispiel 1 in einem Puffer bei pH 5,0 und 37°C betrug nach 2 Stunden 4,8%, nach 4 Stunden 10,3%, jeweils bezogen auf den Gesamtgehalt der Partikel an Naturstoff. Durch Verwendung einer Pufferlösung mit Lipase (Lipomod 34P; die Konzentration des Lipomods 34P betrug 0.5 mg/ml) erhöhen sich diese Werte auf 11,4% nach 2 Stunden bzw. 56,4% nach 4 Stunden.

Überraschend wurde gefunden, dass die Freisetzung des bioaktiven Wirkstoffs aus den Partikeln durch den Zusatz eines Glycerids mit einem Schmelzpunkt von mindestens 35°C erheblich verlangsamt wird, während der enzymatische Abbau des Verkapselungsmaterials beschleunigt wird. Hierdurch kann die Freisetzung des Wirkstoffes besonders gezielt, beispielsweise auf menschlicher Haut erfolgen.

**Patentansprüche**

1. Präparat umfassend mindestens ein Verkapselungsmaterial und mindestens einen bioaktiven Naturstoff, wobei der bioaktive Naturstoff gesteuert aus dem Präparat freigesetzt werden kann, **dadurch gekennzeichnet, dass** das Verkapselungsmaterial mindestens ein Triglycerid mit einem Schmelzpunkt von mindestens 35°C, das von Carbonsäuren mit 12 bis 24 Kohlenstoffatomen abgeleitet ist, und zusätzlich mindestens ein Polymer mit Polyestereinheiten umfasst, wobei das Polymer mit Polyestereinheiten eine Schmelztemperatur von mindestens 30°C und eine Viskosität im Bereich von 50 mPa*s bis 250 Pa*s, gemessen mittels Rotationsviskosimetrie bei 110°C, aufweist.

2. Präparat gemäß Anspruch 1, **dadurch gekennzeichnet, dass** der bioaktive Naturstoff in dem Verkapselungsmaterial homogen dispergiert ist.

3. Präparat gemäß Anspruch 1 oder 2, **dadurch gekennzeichnet, dass** der bioaktive Naturstoff in dem Verkapselungsmaterial gelöst vorliegt.

4. Präparat gemäß einem der vorhergehenden Ansprüche, **dadurch gekennzeichnet, dass** der Beladungsgrad in einem Bereich von 1% bis 95%, liegt, wobei der Beladungsgrad durch den Gewichtsanteil der freizusetzenden Substanz am Gesamtgewicht des Präparats gegeben ist.

5. Präparat gemäß einem der vorhergehenden Ansprüche, **dadurch gekennzeichnet, dass** das Polymer mit Polyestereinheiten eine Hydroxyzahl im Bereich von 0 bis 200 mg KOH/g aufweist.

6. Präparat gemäß einem der vorhergehenden Ansprüche, **dadurch gekennzeichnet, dass** das Polymer mit Polyestereinheiten eine Schmelztemperatur von mindestens 35°C aufweist.

7. Präparat gemäß einem der vorhergehenden Ansprüche, **dadurch gekennzeichnet, dass** das Polymer mit Polyestereinheiten eine Säurezahl im Bereich von 0 bis 20 mg KOH/g aufweist.

8. Präparat gemäß einem der vorhergehenden Ansprüche, **dadurch gekennzeichnet, dass** das Polymer mit Polyestereinheiten ein Molekulargewicht im Bereich von 1000 g/mol bis 400000 g/mol aufweist.

9. Präparat gemäß einem der vorhergehenden Ansprüche, **dadurch gekennzeichnet, dass** das Polymer mit Polyestereinheiten eine Viskosität im Bereich von 100 mPa*s bis 100 Pa*s aufweist, gemessen mittels Rotationsviskosimetrie bei 110°C.

10. Präparat gemäß einem der vorhergehenden Ansprüche, **dadurch gekennzeichnet, dass** das Triglycerid einen Schmelzpunkt im Bereich von 45 bis 80°C aufweist.

11. Präparat gemäß einem der vorhergehenden Ansprüche, **dadurch gekennzeichnet, dass** das Triglycerid eine dynamische Viskosität im Bereich von 10 bis 50 mPa*s bei 70°C aufweist

12. Präparat gemäß einem der vorhergehenden Ansprüche, **dadurch gekennzeichnet, dass** das Polymer mit Polyestereinheiten ein hyperverzweigtes Polymer ist, das einen hydrophilen Kern mit Polyestereinheiten sowie hydrophobe Endgruppen umfasst.

13. Präparat gemäß Anspruch 12, **dadurch gekennzeichnet, dass** das hyperverzweigte Polymer ein Molekulargewicht größer oder gleich 3000 g/mol und eine Hydroxyzahl im Bereich von 0 bis 200 mg KOH/g aufweist, der Verzweigungsgrad im Bereich von 20 bis 70% liegt und das hyperverzweigte Polymer eine Schmelztemperatur von mindestens 30°C aufweist.

14. Präparat gemäß Anspruch 12 oder 13, **dadurch gekennzeichnet, dass** das hyperverzweigte Polymer einen Funktionalisierungsgrad von mindestens 5% aufweist.

15. Präparat gemäß einem der Ansprüche 12 bis 14, **dadurch gekennzeichnet, dass** der hydrophile Kern mindestens 90 Gew.-% an Wiederholungseinheiten aufweist, die von Polyester bildenden Monomeren abgeleitet sind.

16. Präparat gemäß einem der Ansprüche 12 bis 15, **dadurch gekennzeichnet, dass** der hydrophile Kern eine zentrale Einheit, die von einem Initiatormolekül mit mindestens zwei Hydoxygruppen abgeleitet ist, und Wiederholungseinheiten, die von Monomeren mit mindestens einer Carboxylgruppe und mindestens zwei Hydroxygruppen abgeleitet sind, aufweist.

17. Präparat gemäß einem der Ansprüche 12 bis 16, **dadurch gekennzeichnet, dass** die hydrophoben Endgruppen durch Gruppen gebildet werden, die von Carbonsäuren mit mindestens 10 Kohlenstoffatomen abgeleitet sind.

18. Präparat gemäß einem der Ansprüche 12 bis 17, **dadurch gekennzeichnet, dass** mindestens ein Teil der hydrophoben Endgruppen durch Gruppen gebildet werden, die von Carbonsäuren mit höchstens 18 Kohlenstoffatomen abgeleitet sind.

19. Präparat gemäß Anspruch 17 oder 18, **dadurch gekennzeichnet, dass** der Anteil der Carbonsäuren mit 12 bis 18 Kohlenstoffatomen mindestens 30 Gew.-%, bezogen auf das Gewicht der zur Hydrophobisierung eingesetzten Carbonsäuren, beträgt.

20. Präparat gemäß einem der vorhergehenden Ansprüche, **dadurch gekennzeichnet, dass** das Gewichtsverhältnis von Triglycerid zu Polymer mit Polyestereinheiten im Bereich von 10:1 bis 1:10 liegt.

21. Präparat gemäß einem der vorhergehenden Ansprüche, **dadurch gekennzeichnet, dass** der bioaktive Naturstoff mindestens eine Verbindung ausgewählt aus der Gruppe bestehend aus Tocopherol und Derivate, Ascorbinsäure und Derivate, Desoxyribonucleinsäure, Retinol und Derivate, Alpha-Liponsäure, Niacinamid, Ubichinon, Bisabolol, Allantoin, Phytantriol, Panthenol, AHA-Säuren, Aminosäuren, Hyaluronsäure, Polyglutaminsäure, Beta-Glucane, Creatin und Creatinderivate, Guanidin und Guanidinderivate, Ceramide, Sphingolipide, Phytosphingosin und Phytosphingosinderivate, Sphingosin und Sphingosinderivate, Sphinganin und Sphinganinderivate, Pseudo-Ceramiden, essentiellen Ölen, Peptiden, Proteinen, Proteinhydrolysaten, Pflanzenextrakten und Vitaminkomplexen umfasst oder der bioaktive Naturstoff ein Geschmacksstoff, ein Aromastoff, ein natürliches Extrakt, eine geschmacksverstärkende Verbindung, ein natürliches Wachs, ein Protein, ein Peptid, ein Vitamin, eine Vitaminvorstufe, ein Fett, eine Fettsäure, eine Aminosäure, eine Aminosäurevorstufe, ein Zucker, ein Zuckerderivat, ein Nukleotid oder eine Nukleinsäure sowie Vorstufen und Derivate derselben, ein Arzneimittel, ein Enzym, ein Coenzym oder eine Mischung

dieser Verbindungen oder ein Kernöl ist.

**22.** Präparat gemäß einem der vorhergehenden Ansprüche, **dadurch gekennzeichnet, dass** das Verkapselungsmaterial enzymatisch abbaubar ist.

**23.** Präparat gemäß einem der vorhergehenden Ansprüche, **dadurch gekennzeichnet, dass** das Präparat partikelförmig ist und mindestens 80 Gew.-% der Partikel innerhalb eines Größenbereichs von 1 μm bis 100 μm liegen.

**24.** Verwendung eines Präparats gemäß mindestens einem der Ansprüche 1 bis 22 in Kosmetika, in Deodorants, in Nahrungsmitteln, in Futtermitteln, in Getränken, in Emollients, in Phytonutrients und/oder in Verpackungen.

**Claims**

**1.** Preparation comprising at least one encapsulation material and at least one bioactive natural substance, which bioactive natural substance can be released from the preparation in a controlled manner, **characterized in that** the encapsulation material comprises at least one triglyceride having a melting point of at least 35°C and being derived from carboxylic acids having 12 to 24 carbon atoms, and additionally at least one polymer with polyester units, said polymer with polyester units having a melting temperature of at least 30°C and a viscosity in the range from 50 mPa*s to 250 Pa*s, measured by means of rotational viscometry at 110°C.

**2.** Preparation according to Claim 1, **characterized in that** the bioactive natural substance is dispersed homogeneously in the encapsulation material.

**3.** Preparation according to Claim 1 or 2, **characterized in that** the bioactive natural substance is present dissolved in the encapsulation material.

**4.** Preparation according to any one of the preceding claims, **characterized in that** the degree of loading is within a range from 1% to 95%, the degree of loading being given by the proportion by weight of the substance to be released in the total weight of the preparation.

**5.** Preparation according to any one of the preceding claims, **characterized in that** the polymer with polyester units has a hydroxyl number in the range from 0 to 200 mg KOH/g.

**6.** Preparation according to any one of the preceding claims, **characterized in that** the polymer with polyester units has a melting temperature of at least 35°C.

**7.** Preparation according to any one of the preceding claims, **characterized in that** the polymer with polyester units has an acid number in the range from 0 to 20 mg KOH/g.

**8.** Preparation according to any one of the preceding claims, **characterized in that** the polymer with polyester units has a molecular weight in the range from 1000 g/mol to 400 000 g/mol.

**9.** Preparation according to any one of the preceding claims, **characterized in that** the polymer with polyester units has a viscosity in the range from 100 mPa*s to 100 Pa*s, measured by means of rotational viscometry at 110°C.

**10.** Preparation according to any one of the preceding claims, **characterized in that** the triglyceride has a melting point in the range from 45 to 80°C.

**11.** Preparation according to any one of the preceding claims, **characterized in that** the triglyceride has a dynamic viscosity in the range from 10 to 50 mPa*s at 70°C.

**12.** Preparation according to any one of the preceding claims, **characterized in that** the polymer with polyester units is a hyperbranched polymer which comprises a hydrophilic core with polyester units and hydrophobic end groups.

**13.** Preparation according to Claim 12, **characterized in that** the hyperbranched polymer has a molecular weight greater than or equal to 3000 g/mol and a hydroxyl number in the range from 0 to 200 mg KOH/g, the degree of branching is in the range from 20 to 70% and the hyperbranched polymer has a melting temperature of at least 30°C.

**14.** Preparation according to Claim 12 or 13, **characterized in that** the hyperbranched polymer has a degree of functionalization of at least 5%.

**15.** Preparation according to any one of Claims 12 to 14, **characterized in that** the hydrophilic core has at least 90% by weight of repeat units derived from polyester-forming monomers.

**16.** Preparation according to any one of Claims 12 to 15, **characterized in that** the hydrophilic core has a central unit which is derived from an initiator molecule having at least two hydroxyl groups, and repeat units which are derived from monomers having at least one carboxyl group and at least two hydroxyl groups.

**17.** Preparation according to any one of Claims 12 to 16, **characterized in that** the hydrophobic end groups are formed by groups derived from carboxylic acids having at least 10 carbon atoms.

**18.** Preparation according to any one of Claims 12 to 17, **characterized in that** at least some of the hydrophobic end groups are formed by groups derived from carboxylic acids having at most 18 carbon atoms.

**19.** Preparation according to Claim 17 or 18, **characterized in that** the proportion of the carboxylic acids having 12 to 18 carbon atoms is at least 30% by weight, based on the weight of the carboxylic acids used for the hydrophobization.

**20.** Preparation according to any one of the preceding claims, **characterized in that** the weight ratio of triglyceride to polymer with polyester units is in the range from 10:1 to 1:10.

**21.** Preparation according to any one of the preceding claims, **characterized in that** the bioactive natural substance comprises at least one compound selected from the group consisting of tocopherol and derivatives, ascorbic acid and derivatives, deoxyribonucleic acid, retinol and derivatives, alpha-lipoic acid, niacinamide, ubiquinone, bisabolol, allantoin, phytantriol, panthenol, AHA acids, amino acids, hyaluronic acid, polyglutamic acid, beta-glucans, creatin and creatin derivatives, guanidine and guanidine derivatives, ceramides, sphingolipids, phytosphingosine and phytosphingosine derivatives, sphingosine and sphingosine derivatives, sphinganine and sphinganine derivatives, pseudo-ceramides, essential oils, peptides, proteins, protein hydrolysates, plant extracts and vitamin complexes, or the bioactive natural substance is a flavoring, an aroma, a natural extract, a flavor-enhancing compound, a natural wax, a protein, a peptide, a vitamin, a vitamin precursor, a fat, a fatty acid, an amino acid, an amino acid precursor, a sugar, a sugar derivative, a nucleotide or a nucleic acid and precursors and derivatives thereof, a medicament, an enzyme, a coenzyme, or a mixture of said compounds, or a kernel oil.

**22.** Preparation according to any one of the preceding claims, **characterized in that** the encapsulation material is enzymatically degradable.

**23.** Preparation according to any one of the preceding claims, **characterized in that** the preparation is particulate and at least 80% by weight of the particles are within a size range from 1 $\mu$m to 100 $\mu$m.

**24.** Use of a preparation according to at least one of Claims 1 to 22 in cosmetics, in deodorants, in foods, in animal feeds, in drinks, in emollients, in phytonutrients and/or in packagings.

## Revendications

**1.** Préparation comprenant au moins un matériau d'encapsulation et au moins une substance naturelle bioactive, la substance naturelle bioactive pouvant être libérée de manière contrôlée de la préparation, **caractérisée en ce que** le matériau d'encapsulation comprend au moins un triglycéride présentant un point de fusion d'au moins 35°C, qui est dérivé d'acides carboxyliques comprenant 12 à 24 atomes de carbone et en outre au moins un polymère présentant des unités polyester, le polymère présentant des unités polyester présentant une température de fusion d'au moins 30°C et une viscosité dans la plage de 50 mPa.s à 250 Pa.s, mesurée par viscosimétrie par rotation à 110°C.

**2.** Préparation selon la revendication 1, **caractérisée en ce que** la substance naturelle bioactive est dispersée de manière homogène dans le matériau d'encapsulation.

**3.** Préparation selon la revendication 1 ou 2, **caractérisée en ce que** la substance naturelle bioactive se trouve sous

forme dissoute dans le matériau d'encapsulation.

4. Préparation selon l'une quelconque des revendications précédentes, **caractérisée en ce que** le degré de chargement se situe dans une plage de 1% à 95%, le degré de chargement étant donné par la proportion pondérale de la substance à libérer par rapport au poids total de la préparation.

5. Préparation selon l'une quelconque des revendications précédentes, **caractérisée en ce que** le polymère présentant des unités polyester présente un indice d'hydroxy dans la plage de 0 à 200 mg de KOH/g.

6. Préparation selon l'une quelconque des revendications précédentes, **caractérisée en ce que** le polymère présentant des unités polyester présente une température de fusion d'au moins 35°C.

7. Préparation selon l'une quelconque des revendications précédentes, **caractérisée en ce que** le polymère présentant des unités polyester présente un indice d'acide dans la plage de 0 à 20 mg de KOH/g.

8. Préparation selon l'une quelconque des revendications précédentes, **caractérisée en ce que** le polymère présentant des unités polyester présente un poids moléculaire dans la plage de 1000 g/mole à 400 000 g/mole.

9. Préparation selon l'une quelconque des revendications précédentes, **caractérisée en ce que** le polymère présentant des unités polyester présente une viscosité dans la plage de 100 mPa.s à 100 Pa.s, mesurée par viscosimétrie par rotation à 110°C.

10. Préparation selon l'une quelconque des revendications précédentes, **caractérisée en ce que** le triglycéride présente un point de fusion dans la plage de 45 à 80°C.

11. Préparation selon l'une quelconque des revendications précédentes, **caractérisée en ce que** le triglycéride présente une viscosité dynamique dans la plage de 10 à 50 mPa.s à 70°C.

12. Préparation selon l'une quelconque des revendications précédentes, **caractérisée en ce que** le polymère présentant des unités polyester est un polymère hyperramifié, qui comprend un noyau hydrophile présentant des unités polyester ainsi que des groupes terminaux hydrophobes.

13. Préparation selon la revendication 12, **caractérisée en ce que** le polymère hyperramifié présente un poids moléculaire supérieur ou égal à 3000 g/mole et un indice d'hydroxyle dans la plage de 0 à 200 mg de KOH/g, le degré de ramification se situe dans la plage de 20 à 70% et le polymère hyperramifié présente une température de fusion d'au moins 30°C.

14. Préparation selon la revendication 12 ou 13, **caractérisée en ce que** le polymère hyperramifié présente un degré de fonctionnalisation d'au moins 5%.

15. Préparation selon l'une quelconque des revendications 12 à 14, **caractérisée en ce que** le noyau hydrophile présente au moins 90% en poids d'unités répétitives qui sont dérivées de monomères formant le polyester.

16. Préparation selon l'une quelconque des revendications 12 à 15, **caractérisée en ce que** le noyau hydrophile présente une unité centrale qui est dérivée d'une molécule d'initiateur comprenant au moins deux groupes hydroxy, et des unités répétitives qui sont dérivées de monomères présentant au moins un groupe carboxyle et au moins deux groupes hydroxy.

17. Préparation selon l'une quelconque des revendications 12 à 16, **caractérisée en ce que** les groupes terminaux hydrophobes sont formés par des groupes dérivés d'acides carboxyliques comprenant au moins 10 atomes de carbone.

18. Préparation selon l'une quelconque des revendications 12 à 17, **caractérisée en ce qu'**au moins une partie des groupes terminaux hydrophobes est formée par des groupes dérivés d'acides carboxyliques comprenant au plus 18 atomes de carbone.

19. Préparation selon la revendication 17 ou 18, **caractérisée en ce que** la proportion des acides carboxyliques comprenant 12 à 18 atomes de carbone représente au moins 30% en poids, par rapport au poids des acides carboxyliques

utilisés pour l'hydrofugation.

**20.** Préparation selon l'une quelconque des revendications précédentes, **caractérisée en ce que** le rapport pondéral du triglycéride au polymère présentant des unités polyester se situe dans la plage de 10:1 à 1:10.

**21.** Préparation selon l'une quelconque des revendications précédentes, **caractérisée en ce que** la substance naturelle bioactive comprend au moins un composé choisi dans le groupe constitué par le tocophérol et ses dérivés, l'acide ascorbique et ses dérivés, l'acide désoxyribonucléique, le rétinol et ses dérivés, l'acide alpha-liponique, le niacinamide, l'ubiquinone, le bisabolol, l'allantoïne, le phytantriol, le panthénol, les acides AHA (alpha-hydroxyacides), les acides aminés, l'acide hyaluronique, l'acide polyglutamique, le bêta-glucane, la créatine et les dérivés de créatine, la guanidine et les dérivés de guanidine, les céramides, les sphingolipides, la phytosphingosine et les dérivés de phytosphingosine, la sphingosine et les dérivés de sphingosine, la sphinganine et les dérivés de sphinganine, les pseudo-céramides, les huiles essentielles, les peptides, les protéines, les hydrolysats de protéines, les extraits végétaux et les complexes vitaminés ou la substance naturelle bioactive est un agent gustatif, un arôme, un extrait naturel, un composé exhausteur de goût, une cire naturelle, une protéine, un peptide, une vitamine, un précurseur de vitamine, une graisse, un acide gras, un acide aminé, un précurseur d'acide aminé, un sucre, un dérivé de sucre, un nucléotide ou un acide nucléique ainsi que des précurseurs et des dérivés de ceux-ci, un médicament, une enzyme, une coenzyme ou un mélange de ces composés ou une huile de noyau.

**22.** Préparation selon l'une quelconque des revendications précédentes, **caractérisée en ce que** le matériau d'encapsulation est dégradable par voie enzymatique.

**23.** Préparation selon l'une quelconque des revendications précédentes, **caractérisée en ce que** la préparation est particulaire et au moins 80% en poids des particules se situent dans une plage de grosseurs de 1 μm à 100 μm.

**24.** Utilisation d'une préparation selon au moins l'une quelconque des revendications 1 à 22 dans des cosmétiques, des déodorants, des aliments, des fourrages, des boissons, des émollients, des phytonutriments et/ou dans des emballages.

Fig. 1

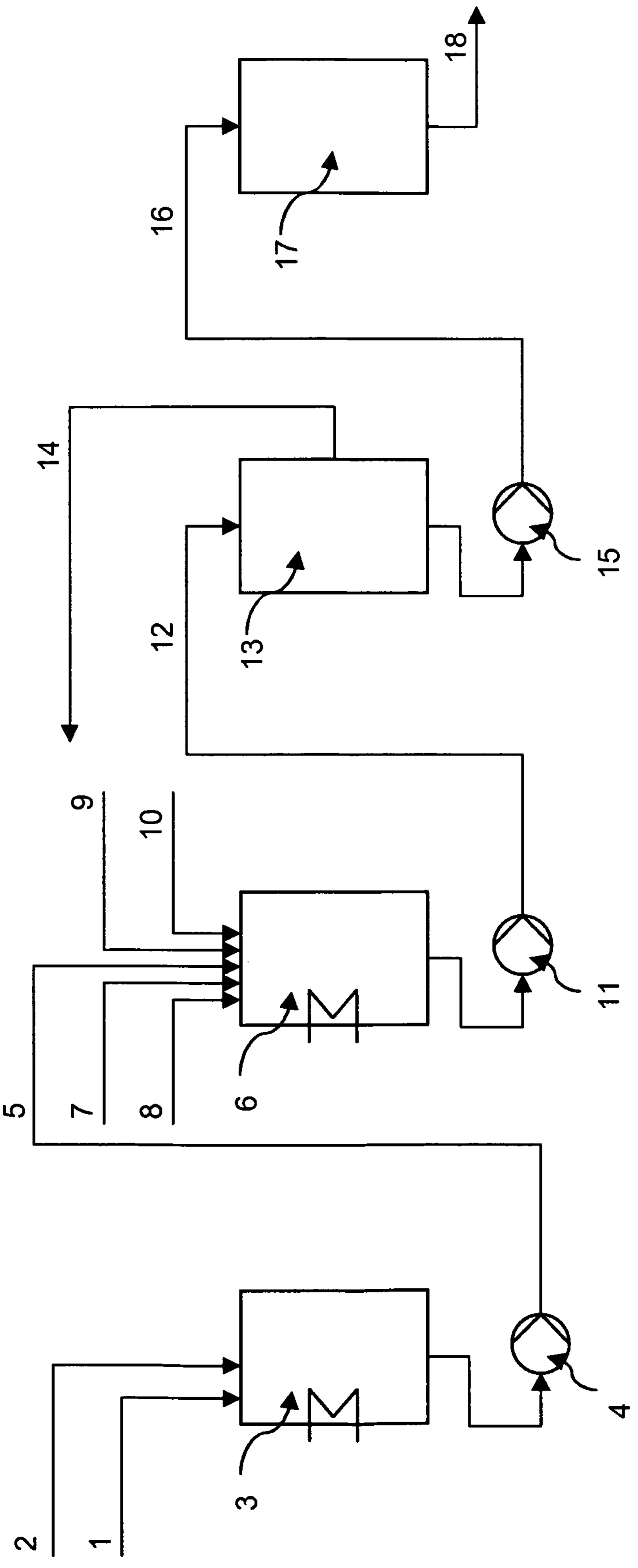

Fig. 2

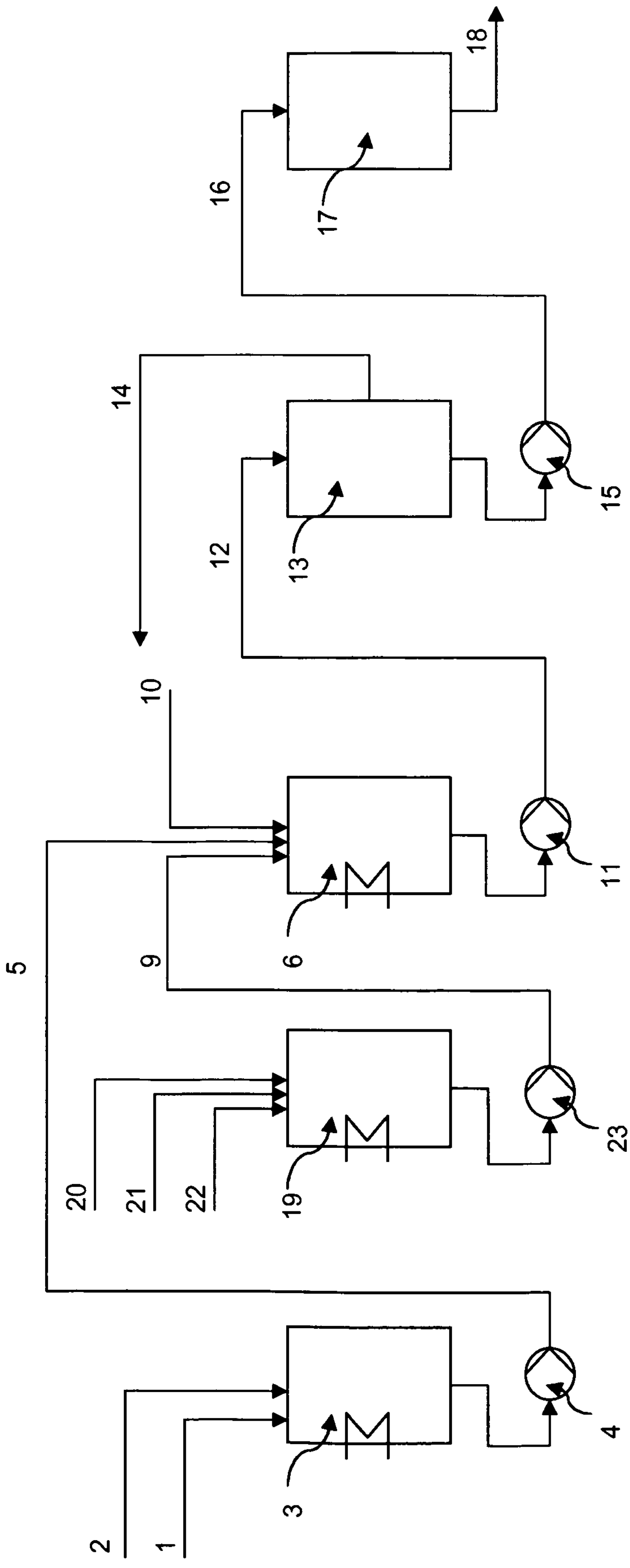

Fig. 3

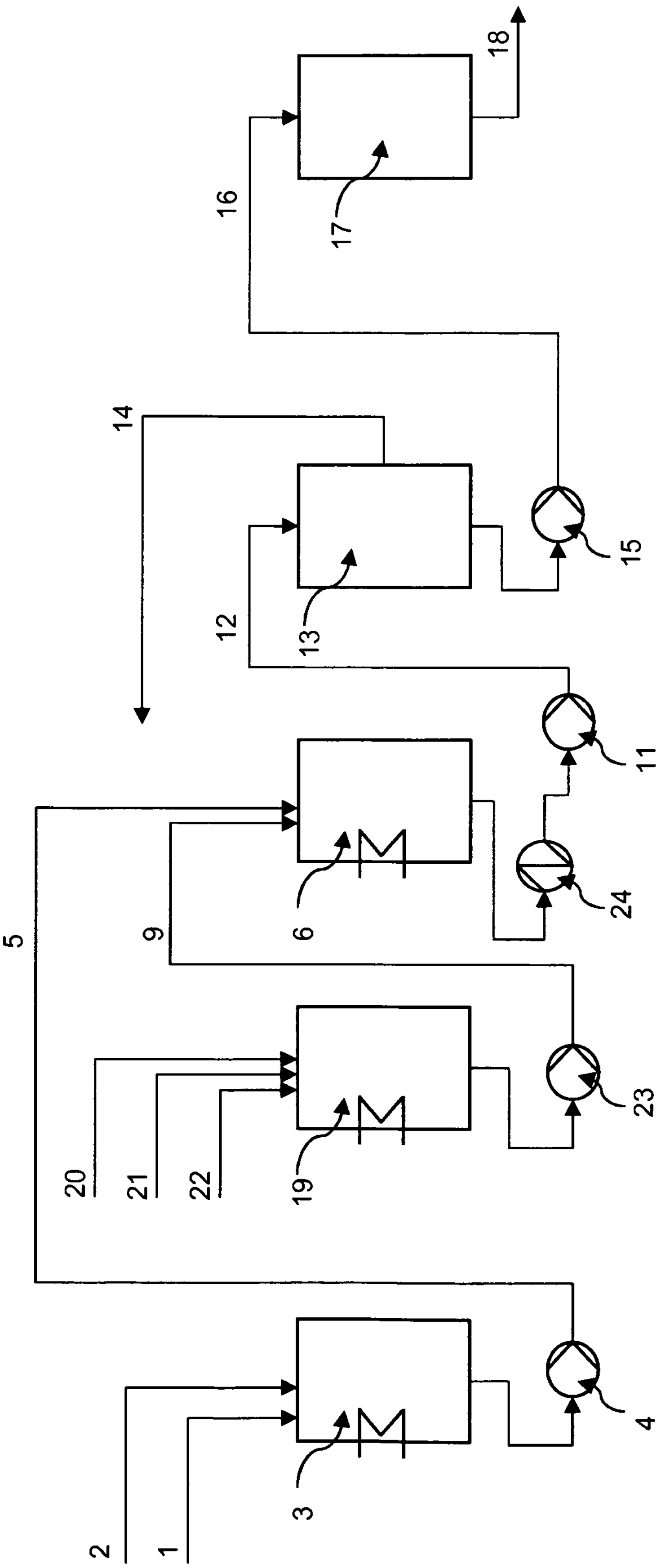

Fig. 4

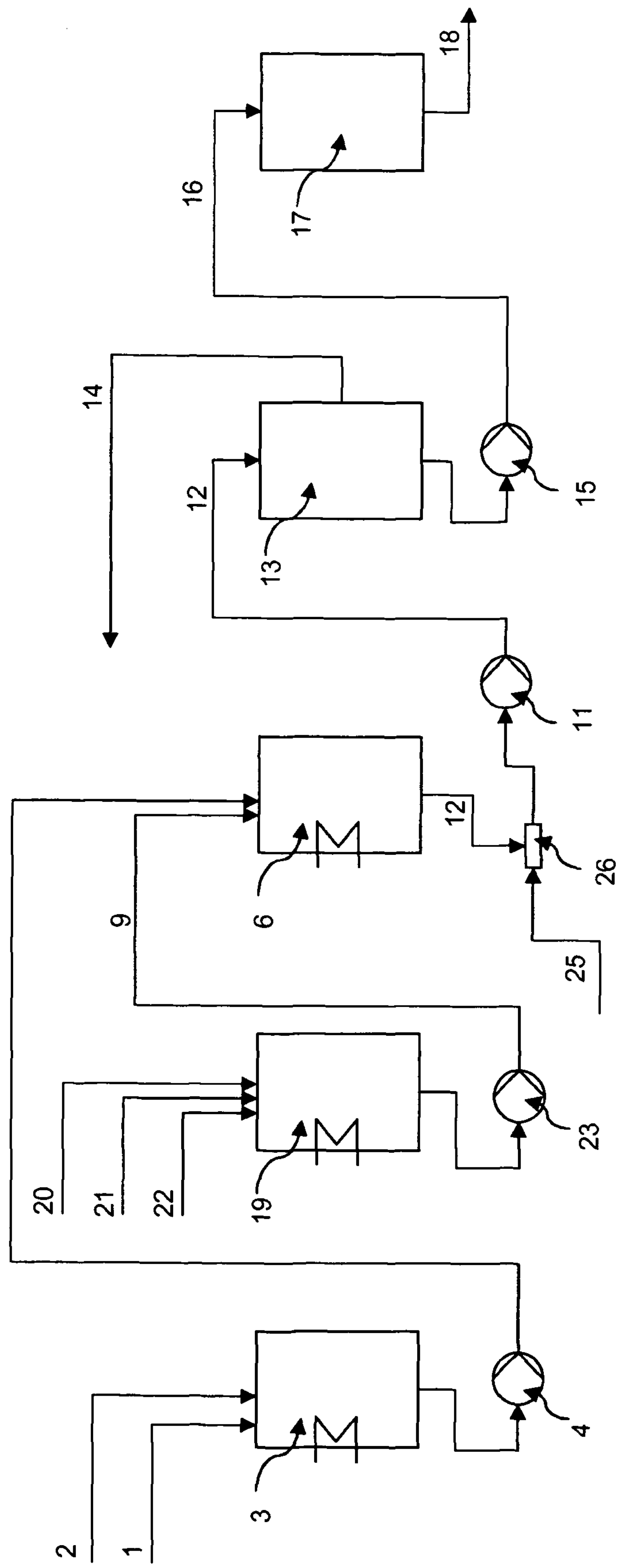

Fig. 5

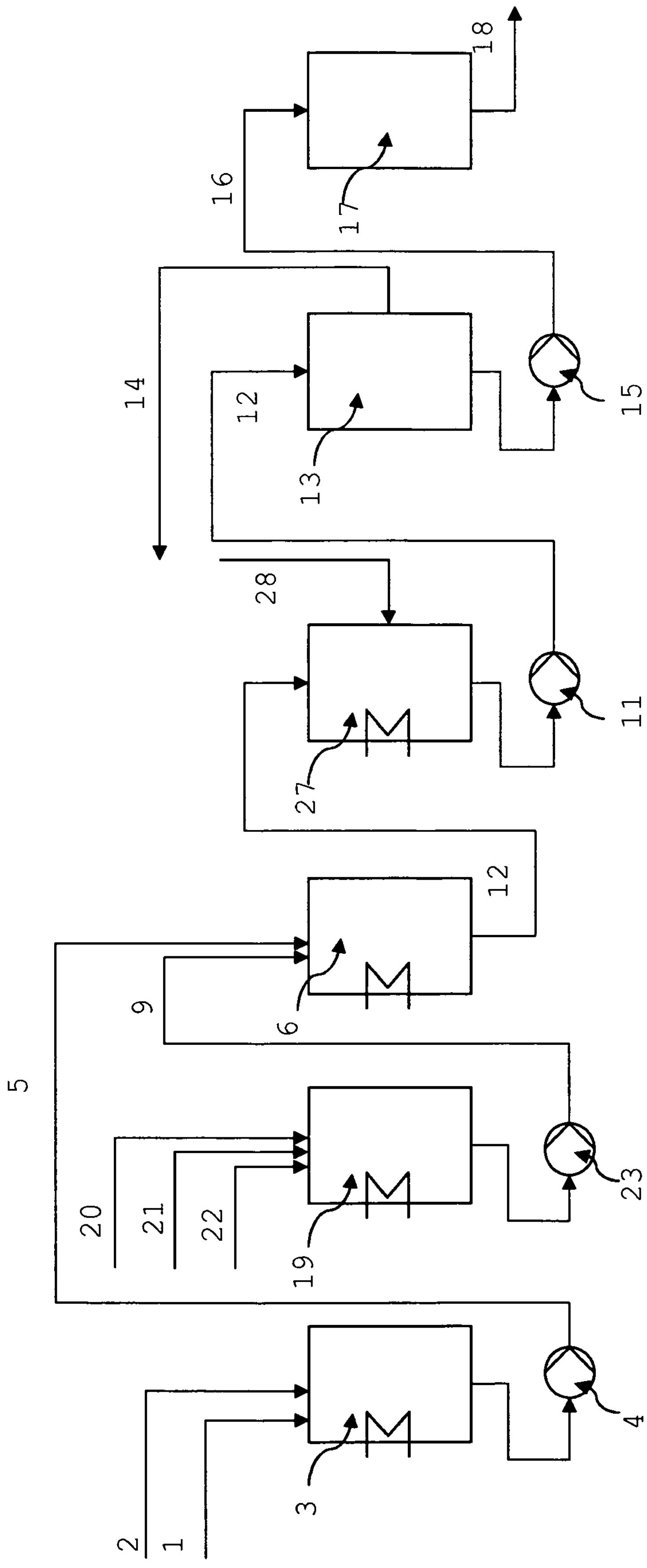

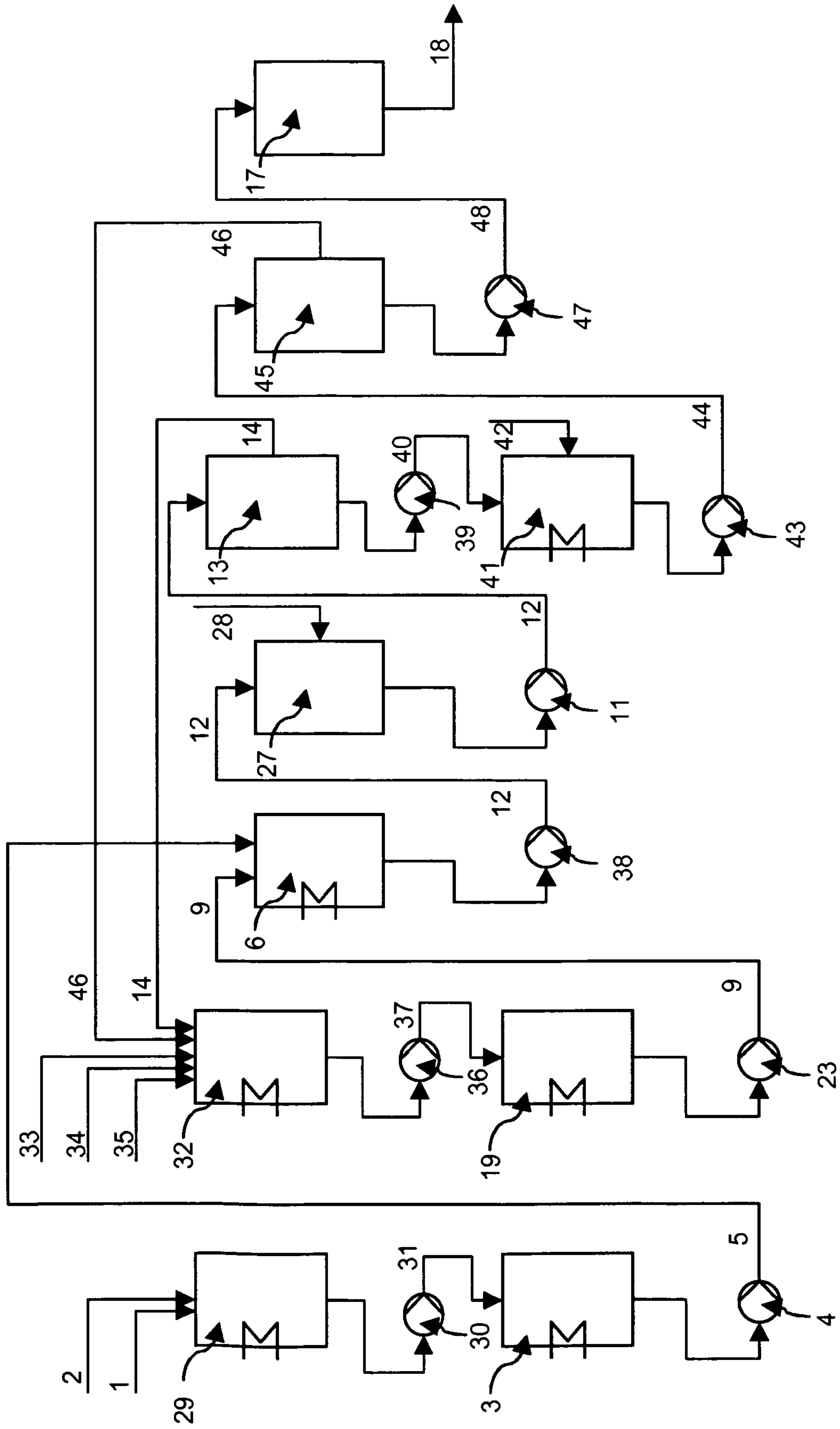

Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004072153 A
- WO 00065024 A
- WO 2005034909 A
- WO 03037383 A
- WO 0006267 A
- WO 03033027 A
- US 6379683 B
- EP 1034839 B1
- US 6432423 B
- US 6475495 B
- US 20060030686 A
- WO 2006047714 A
- WO 2004028269 A
- US 2004016394 A
- EP 0630389 A
- EP 630389 A
- DE 102005037209 A
- DE 102005037210 A
- WO 9317060 A


### In der Beschreibung aufgeführte Nicht-Patentliteratur


- **ZOU et al.** *Macromol. Biosci,* 2005, vol. 5, 662-668
- **BURGATH.** *Macromol.Chem. Phys.,* 2000, vol. 201, 782-791
- **D.HÖLTER ; A.BURGATH ; H.FREY.** *Acta Polymer,* 1997, vol. 48, 30
- **H. MAGNUSSON ; E. MALMSTRÖM ; A. HULT ; M. JOANSSON.** *Polymer,* 2002, vol. 43, 301
- **SMIRNOVA, I. ; SUTTIRUENGWONG, S. ; ARLT, W.** Feasibility study of hydrophilic and hydrophobic silica aerogels as drug delivery systems. *Journal of Non-Crystalline Solids,* 2004, 54-60
- **GAMSE et al.** *Chemie Ingenieur Technik,* 2005, vol. 77 (6), 669-679